(19) Europäisches Patentamt
European Patent Office
Office européen des brevets

(11) **EP 1 803 404 B1**

(12) **EUROPEAN PATENT SPECIFICATION**

(45) Date of publication and mention
of the grant of the patent:
**26.02.2014  Bulletin 2014/09**

(51) Int Cl.:
*A61B 8/12* (2006.01)      *A61B 8/08* (2006.01)

(21) Application number: **05793702.1**

(22) Date of filing: **11.10.2005**

(86) International application number:
**PCT/JP2005/018677**

(87) International publication number:
**WO 2006/041050 (20.04.2006 Gazette 2006/16)**

(54) **ULTRASONIC PROBE OF TYPE TO BE INSERTED IN BODY, AND ULTRASONIC IMAGING DEVICE**

ULTRASCHALLSONDE ZUR EINFÜHRUNG IN DEN KÖRPER UND ULTRASCHALLDARSTELLUNGSVORRICHTUNG

SONDE A ULTRASONS APPLIQUEE SUR UN CORPS, ET DISPOSITIF D IMAGERIE A ULTRASONS

(84) Designated Contracting States:
**DE FR GB IT NL**

(30) Priority: **12.10.2004   JP 2004297340**
**30.11.2004   JP 2004345670**

(43) Date of publication of application:
**04.07.2007   Bulletin 2007/27**

(73) Proprietor: **Hitachi Medical Corporation**
**Tokyo 101-0021 (JP)**

(72) Inventor: **MATSUMURA, Takeshi**
**2701166 (JP)**

(74) Representative: **Strehl Schübel-Hopf & Partner**
**Maximilianstrasse 54**
**80538 München (DE)**

(56) References cited:
EP-A- 1 629 777          WO-A1-98/13094
WO-A1-2004/105615        JP-A- 6 169 922
US-A1- 2002 068 870      US-A1- 2002 068 870
US-B1- 6 511 427

• SHIINA T ET AL: "REAL TIME TISSUE ELASTICITY IMAGING USING THE COMBINED AUTOCORRELATION METHOD" JOURNAL OF MEDICAL ULTRASONICS, JAPAN SOCIETY OF ULTRASONICS IN MEDICINE, TOKYO, JP, vol. 29, no. AUTUMN, 21 September 2002 (2002-09-21), pages 119-128, XP008033652 ISSN: 1346-4523
• NITTA N. AND SHIINA T.: 'Choonpa ni yoru Soshiki no Hisenkei Dansei Tokusei no Gazoka. (A Visualization of Non-Linear Elasticity Property of Tissues by Ultrasound)' THE TRANSACTIONS OF THE INSTITUTE OF ELECTRONICS, INFORMATION AND COMMUNICATION ENGINEERS A. no. 12, 01 December 2001, pages 1405 - 1413, XP002979103
• MURAYAMA N ET AL: 'EUB-8500 no okeru Real-Time Tissue Elastography Kino no Kaihatsu.' JPN. J. OF MED. ULTRASONICS. vol. 31, 15 April 2004, page S113, XP002995855
• MATSUMURA T ET AL: 'Real time Soshiki Dansei Imaging Systems no Zenritsusen eno Tekiyo.' JOURNAL OF MEDICAL ULTRASONICS. vol. 31, 15 April 2004, page S114, XP002995856

**Description**

TECHNICAL FIELD

The present invention relates to an ultrasonic imaging device that acquires a tomographic image of a region to be imaged within a subject using ultrasonic waves. In particular, the present invention relates to an ultrasonic imaging device which can calculate a distortion and an elastic modulus at each point on an image from a set of temporally-arranged ultrasonic wave reception signal frame data, and display the calculated distortion and elastic modulus as an elasticity image that quantitatively indicates the hardness or softness of biological tissue.

BACKGROUND ART

A conventional, generally-used ultrasonic imaging device includes an ultrasonic wave transmission and reception controlling means for controlling the transmission and reception of ultrasonic waves; ultrasonic wave transmitting and receiving means for transmitting ultrasonic waves to a subject and receiving the ultrasonic waves from the subject; tomographic scanning means for repeatedly acquiring, at predetermined intervals, data of a tomographic image of the interior of the subject, including a locomotive tissue, by use of a reflection echo signal from the ultrasonic wave transmitting and receiving means; and image displaying means for displaying the time-series tomographic image data acquired by the tomographic scanning means. The structure of the biological tissue within the subject is displayed as, for example, a B-mode image.

In a method which is recently being realized, an external force is manually applied from the body surface of a subject by use of the ultrasonic wave transmitting and receiving surface of an ultrasonic probe. The displacement at each point within the subject is obtained by using the correlation calculation performed on the ultrasonic wave reception signals of two temporally adjacent frames (two consecutive frames). The distortion is further measured through spatial differentiation of the displacement, and the distortion data is converted to an image. Furthermore, in another method which is being realized, data of elastic modulus (e.g., Young's modulus of elasticity) of the body tissue is converted to an image using data on the distribution of stress and distortion caused by an external force. Such an elasticity image based on distortion and elastic modulus data (hereafter, referred to as elasticity frame data) enables measurement and display of the hardness or softness of the body tissue. Examples of such ultrasonic devices include those described in Patent Document 1 and Patent Document 2.

In a method used for calculation of elasticity frame data, one piece of elasticity frame data is formed by a single pair of ultrasonic wave reception signal frame data acquired with a fixed time interval therebetween. The respective image qualities of plural pieces of elasticity image data (particularly distortion image data) acquired by a series of pressing processes depend on the pressing speed at the time when the pair of ultrasonic wave reception signal frame data, forming each piece of elasticity image data is acquired.

In addition, it is known that the pressure increasing amount or the pressure decreasing amount suitable for extracting high-quality elasticity image data is within a range in which a distortion of about 0.5% to 1% is generated in the tissue of interest.

    Patent Document 1: Japanese Patent Application Laid-Open (kokai) No. Heisei 5-317313
    Patent Document 2: Japanese Patent Application Laid-Open (*kokai*) No. 2000-60853

US 6,511,427 discloses an intra-corporeal ultrasonic probe using an inflatable balloon for exerting pressure onto a subject. Another prior art probe of this type is disclosed in US 2002/068870 A1.

In the paper "Real time tissue elasticity imaging using the combined auto-correlation method", Journal of Medical Ultrasonics, Japan Society of Ultrasonics in Medicine, Vol. 29, pages 119 to 128, Shiina et al. discuss an autocorrelation method for modelling the elastic properties of tissues in ultrasonic imaging.

EP 1 629 777 A1 relates to an ultrasonic device with similar elements as the present invention. This document has, however, been published after the filing date of the present application.

DISCLOSURE OF THE INVENTION

PROBLEMS TO BE SOLVED BY THE INVENTION

Document JP 6169922 A discloses an intra-corporeal ultrasonic probe having a contact surface which extends in parallel to an insertion direction and which is adapted to come into contact with the subject. The probe comprises pressing means for pressing against a to-be-imaged portion of the subject with a predetermined pressure at the contact surface in a direction perpendicular to the contact surface by feeding liquid to a balloon and removing liquid therefrom. Present claim 1 has been drafted in the two part form in view if this document.

In the elasticity imaging method according to a conventional ultrasonic imaging device, a method in which the tissue of interest is pressed by hand is used. Therefore, it is difficult to continuously press within a range of pressing speed that is suitable for increasing image quality at all times during a series of pressing processes. In addition, since the pressing speed at each time is not fixed, the outputted plurality of elasticity image data are temporally discontinuous, so that a resultant image has a missing frame between the frames of the elasticity image data, which makes image diagnosis difficult to perform.

Furthermore, hand shaking during the pressing process cannot be avoided, and the pressing direction changes at each time, which is also a cause of the discontinuity of the above-described continuously acquired elasticity image data. In addition, the quality of the elasticity image is unavoidably dependant on the skill of the technician.

The present invention has been accomplished in light of the above issues, and an object of the present invention is to provide an ultrasonic probe with improved pressure control for the operator and an ultrasonic imaging device which can stably convert a high-quality elasticity image to a video picture at arbitrary times during elasticity image diagnosis.

MEANS FOR SOLVING THE PROBLEMS

The object is wet by the probe of claim 1. The subclaims relate to preferred embodiments. According to a first example useful to understand the present invention, there is provided an ultrasonic probe for an ultrasonic diagnostic apparatus which acquires an elasticity image while pressing a subject, the ultrasonic probe comprising a pressing member provided on the ultrasonic probe and having a contact surface which extends perpendicular to a pressing direction and which comes into contact with the subject; and pressing means for pressing a to-be-imaged portion of the subject with a predetermined pressure by moving the contact surface in the pressing direction.

By use of an ultrasonic probe including pressing means as in the example, the pressing stage can be moved in the pressing direction at a desired fixed speed. A high-quality elasticity image data can be acquired at an arbitrary time. Furthermore, since repeatability of the pressing action can be maintained, the problem of the quality of the elasticity image being dependant on the technician can be avoided.

According to a second example useful to understand the present invention, there is provided an intra-corporeal ultrasonic probe which is inserted into a subject, comprising a pressing member provided on the probe and having a contact surface which extends parallel to an insertion direction and which comes into contact with the subject; and pressing means for pressing a to-be-imaged portion of the subject with a predetermined pressure at the contact surface in a direction perpendicular to the contact surface.

The intra-corporeal ultrasonic probe according to the second example includes pressing means, as does the ultrasonic probe according to the first example. Whereas the ultrasonic probe according to the first example performs pressing in the moving direction. The intra-corporeal ultrasonic probe according to the second example performs pressing in a direction perpendicular to the insertion direction.

According to a third example useful to understand the present invention, there is provided an ultrasonic imaging device comprising ultrasonic wave transmitting and receiving means for transmitting ultrasonic waves to a subject and receiving ultrasonic waves from the subject by use of the above-described ultrasonic probe; ultrasonic wave transmission and reception control means for controlling the transmission and reception of the ultrasonic waves; tomographic scanning means for repeatedly acquiring, at predetermined intervals, ultrasonic wave reception signal frame data representing an image of the interior of the subject, including a locomotive tissue, by use of a reflection echo signal output from the ultrasonic wave transmitting and receiving means; signal processing means for performing signal processing on a plurality of time-series ultrasonic wave reception signal frame data acquired by the tomographic scanning means; black-and-white brightness information converting means for converting the time-series tomographic frame data from the signal processing means to black-and-white tomographic data; ultrasonic wave reception signal frame data selecting means for selecting a pair of ultrasonic wave reception signal frame data to be subjected to displacement measurement, among the group of time-series ultrasonic wave reception signal frame data acquired by the tomographic scanning means; displacement measuring means for measuring the amount of movement or displacement of each point on a tomographic image, based on the pair of ultrasonic wave reception signal frame data selected by the ultrasonic wave reception signal frame data selecting means; pressure measuring means for measuring or estimating an internal pressure of the to-be-imaged portion of the subject; distortion and elastic modulus calculating means for calculating a distortion and an elastic modulus at each point on the tomographic image, based on the displacement measured by the displacement measuring means and the internal pressure measured by the pressure measuring means, and for generating a first elasticity frame data; elasticity data processing means for performing signal processing on the first elasticity frame data generated by the distortion and elastic modulus calculating means and for generating a second elasticity frame data; color information converting means, or black-and-white brightness information converting mean, for receiving the second elasticity frame data generated by the elasticity data processing means, and for providing color information, or black-and-white brightness information; switching-adding means for receiving the black-and-white tomographic image data from the black- and-white brightness information converting means and the colored elasticity image data from the color information converting means or the black-and-white elasticity image data from the black-and-white brightness information converting means and for outputting them after adding them together or outputting them independently; and image displaying means for displaying the image data from the switching-adding means.

This example is related to an ultrasonic imaging device which can stably convert a high-quality elasticity image to a video picture at arbitrary times by use of the above-described ultrasonic probe.

According to a fourth example useful to understand the present invention, there is provided an intra-corporeal ultrasonic imaging device comprising ultrasonic wave transmitting and receiving means for transmitting ultrasonic waves to a subject and receiving ultrasonic waves from the subject by use of the above-described intra-corporeal ultrasonic probe; ultrasonic wave transmission and reception control means for controlling the transmission and reception of the ultrasonic waves; tomographic scanning means for repeatedly acquiring, at predetermined intervals, ultrasonic wave reception signal frame data representing an image of the interior of the subject, including a locomotive tissue, by use of a reflection echo signal output from the ultrasonic wave transmitting and receiving means; signal processing means for performing signal processing on a plurality of time-series ultrasonic wave reception signal frame data acquired by the tomographic scanning means; black-and-white brightness information converting means for converting the time-series tomographic frame data from the signal processing means to black-and-white tomographic data; ultrasonic wave reception signal frame data selecting means for selecting a pair of ultrasonic wave reception signal frame data to be subjected to displacement measurement, among the group of time-series ultrasonic wave reception signal frame data acquired by the tomographic scanning means; displacement measuring means for measuring the amount of movement or displacement of each point on a tomographic image, based on the pair of ultrasonic wave reception signal frame data selected by the ultrasonic wave reception signal frame data selecting means; pressure measuring means for measuring or estimating an internal pressure of the to-be-imaged portion of the subject; distortion and elastic modulus calculating means for calculating a distortion and an elastic modulus at each point on the tomographic image, based on the displacement measured by the displacement measuring means and the internal pressure measured by the pressure measuring means, and for generating a first elasticity frame data; elasticity data processing means for performing signal processing on the first elasticity frame data generated by the distortion and elastic modulus calculating means and for generating a second elasticity frame data; color information converting means, or black-and-white brightness information converting mean, for receiving the second elasticity frame data generated by the elasticity data processing means, and providing color information, or black-and-white brightness information; switching-adding means for receiving the black-and-white tomographic image data from the black-and-white brightness information converting means and the colored elasticity image data from the color information converting means or the black-and-white elasticity image data from the black-and-white brightness information converting means and for outputting them after adding them together or outputting them independently; and image displaying means for displaying the image data from the switching-adding means.

EFFECTS OF THE INVENTION

According to the present invention, as described above, an effect is achieved in which a high-quality elasticity image can be stably converted to a video picture at an arbitrary time during elasticity image diagnosis.

BRIEF DESCRIPTION OF THE DRAWINGS

[FIG. 1] Block diagram showing an embodiment of an ultrasonic imaging device according to the present invention.
[FIG. 2] Diagram of a generally-used linear ultrasonic probe.
[FIG. 3] Diagram of an ultrasonic probe to which a pressing plate is attached.
[FIG. 4] Diagram of an ultrasonic probe including an automatic pressing mechanism that is a motor mechanism.
[FIG. 5] Diagram of an ultrasonic probe including an automatic pressing mechanism that is a hydraulic mechanism.
[FIG. 6] Diagram of an external-attachment-type automatic pressing mechanism attached to an existing ultrasonic probe.
[FIG. 7] Diagram of an ultrasonic probe to which pressure sensors are attached.
[FIG. 8] Diagram of a pressure measuring section controlling the automatic pressing mechanism based on pressure information.
[FIG. 9] Outside view of a transrectal ultrasonic probe.
[FIG. 10] Partially enlarged view and left-hand side view showing an example of an automatic pressing mechanism provided in the transrectal ultrasonic probe.
[FIG. 11] Partially enlarged view and left-hand side view showing an example of an automatic pressing mechanism provided in the transrectal ultrasonic probe.

[FIG. 12] Partially enlarged view and left-hand side view showing an example of an automatic pressing mechanism provided in the transrectal ultrasonic probe.

[FIG. 13] Partially enlarged view and left-hand side view showing an example of an automatic pressing mechanism provided in the transrectal ultrasonic probe.

[FIG. 14] Partially enlarged view and left-hand side view showing an example of an automatic pressing mechanism provided in the transrectal ultrasonic probe.

[FIG. 15] Partially enlarged view and left-hand side view showing an example of an automatic pressing mechanism provided in the transrectal ultrasonic probe.

[FIG. 16] Partially enlarged view, left-hand side view, and right-hand side view showing an example of an automatic pressing mechanism provided in the transrectal ultrasonic probe.

[FIG. 17] Diagram showing an example operation of a pressing bag described in FIG. 16.

[FIG. 18] Diagram showing another example of the pressing bag.

[FIG. 19] Diagram showing a modification of a driving section that inflates and deflates the pressing bag described in FIG. 13 in accordance with an embodiment of the invention.

[FIG. 20] Partially enlarged view, left-hand side view, and right-hand side view showing a modification of the automatic pressing mechanism provided in the transrectal ultrasonic probe described in FIG. 14.

[FIG. 21] Partially enlarged view, left-hand side view, and right-hand side view showing a modification of the automatic pressing mechanism provided in the transrectal ultrasonic probe in FIG. 20.

[FIG. 22] Partially enlarged view, left-hand side view, and right-hand side view showing a modification of the automatic pressing mechanism provided in the transrectal ultrasonic probe in FIG. 21.

[FIG. 23] Partially enlarged view, left-hand side view, and right-hand side view showing a modification of the automatic pressing mechanism provided in the transrectal ultrasonic probe in FIG. 22.

[FIG. 24] Diagram showing an application of the trigger section of a water pistol as a power source that inserts liquid into the pressing bag and removes liquid from the pressing bag.

[FIG. 25] Diagram showing a modification of FIG. 24 in which the trigger section of a water pistol is integrally provided in the ultrasonic probe and serves as the power source that inserts liquid into the pressing bag and removes liquid from the pressing bag.

[FIG. 26] Diagram showing a modification of FIG. 25 in which, in place of the trigger section of a water pistol, a structure similar to the holding section of a grip dynamometer is integrally provided in the ultrasonic probe and serves as the power source that inserts liquid into the pressing bag and removes liquid from the pressing bag.

[FIG. 27] Diagram showing a modification of FIG. 26 in which a tube from the syringe to the pressing bag is omitted and a pipe incorporated in the probe handle originally attached to the ultrasonic probe is used.

[FIG. 28] Diagram showing a modification of FIG. 24 in which, in place of the water pistol that can be manipulated by fingers, a foot-pedal-type power source that can be operated by foot is used.

DESCRIPTION OF REFERENCE NUMERALS

10: ultrasonic probe
11: ultrasonic wave transmission and reception control circuit
12: wave transmission circuit
13: reception circuit
14: phase adjustment/addition circuit
15: signal processing section
16: black-and-white scan converter
17: switching/adding unit
18: image display unit
19: ultrasonic wave reception signal frame data selecting section
20: displacement measuring section
21: pressure measuring section
22: automatic pressing mechanism
23: distortion and elastic modulus calculating section
24: elasticity data processing section
25: color scan converter
101: ultrasonic wave transmitting and receiving surface
102: pressing stage

103: ultrasonic probe holding section
104: supporting member
105: switch
31: pressing plate
41: motor mechanism
42: gear (pinion)
43: plate gear (rack)
44: motor control section
51, and 51A to 51E: cylinder
511, and 511A to 511E: piston
52, and 52A to 52E: tube
53, and 53A to 53E: pump
55 and 56: pressing bag
57: shell section
60: automatic pressing mechanism
61: ultrasonic probe fixing mechanism
62: supporting member
63: plate gear (rack)
64: driving mechanism (motor mechanism)
65: gear (pinion)
66 and 67: gear
71 to 76: pressure sensor
80: transrectal ultrasonic probe
81: probe gripping section
82: intra-corporeal section
83, and 83A to 83E: pressing bag
84: opening section
85: stopper
86: fixing belt
87: supporting member

BEST MODE FOR CARRYING OUT THE INVENTION

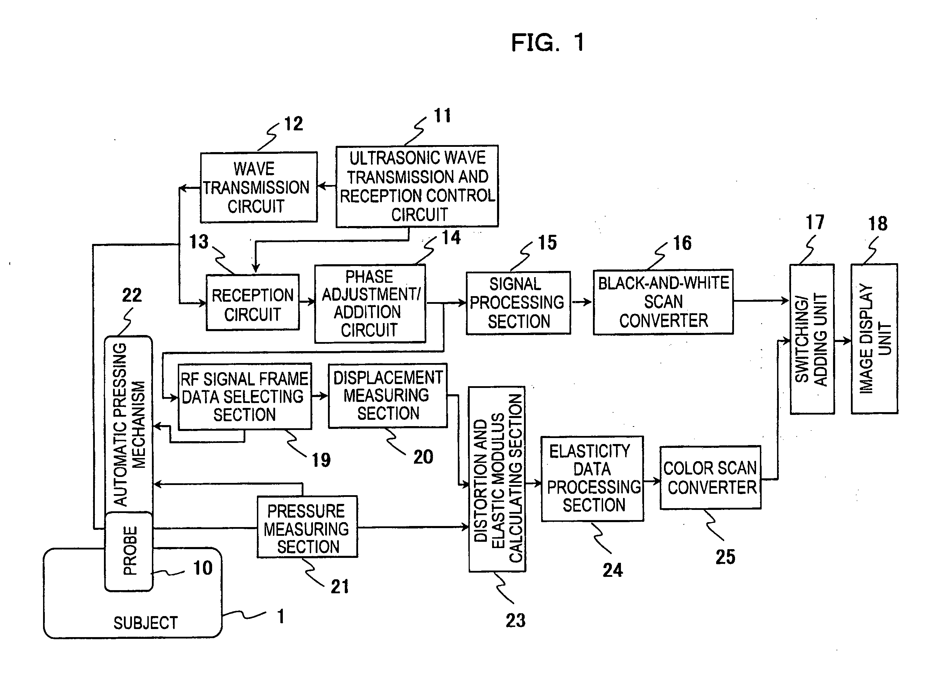

Illustrative examples and embodiments of the present invention will next be described in detail, with reference to the accompanying drawings. FIG. 1 is a block diagram showing an embodiment of an ultrasonic imaging device according to the present invention. The ultrasonic imaging device acquires a tomographic image of a to-be-imaged portion of a subject 1, using ultrasonic waves. In addition, the ultrasonic imaging device displays an elasticity image indicating the hardness of biological tissue. As shown in FIG. 1, the ultrasonic imaging device includes an ultrasonic probe 10, an ultrasonic wave transmission and reception control circuit 11, a wave transmission circuit 12, a reception circuit 13, a phase adjustment/addition circuit 14, a signal processing section 15, a black-and-white scan converter 16, a switching/adding unit 17, an image display unit 18, an ultrasonic wave reception signal frame data selecting section 19, a displacement measuring section 20, a pressure measuring section 21, an automatic pressing mechanism 22, a distortion and elastic modulus calculating section 23, an elasticity data processing section 24, and a color scan converter 25. The ultrasonic probe 10 includes the automatic pressing mechanism 22.

The ultrasonic probe 10 is formed with a plurality of transducers arranged in a strip-like shape. The ultrasonic probe 10 mechanically or electrically performs beam scanning and transmits ultrasonic waves to the subject 1. In addition, the ultrasonic probe 10 receives the ultrasonic waves that are reflected within the subject 1. The ultrasonic probe 10 is brought into contact with the body surface of the subject 1 or is inserted into the subject 1. The ultrasonic probe 10 is configured to apply pressure to a portion of the subject 1 which surrounds the ultrasonic probe 10 in state in which the ultrasonic probe 10 is in contact with the body surface of the subject 1 or inserted into the subject 1. A detailed configuration of the ultrasonic probe 10 will be described hereafter.

The ultrasonic wave transmission and reception control circuit 11 controls the timing at which ultrasonic waves are transmitted and received. The wave transmission circuit 12 generates wave transmission pulses for driving the ultrasonic probe 10 so as to generate the ultrasonic waves. In addition, the wave transmission circuit 12 sets the convergence point of the transmitted ultrasonic waves to a certain depth by means of a wave transmission phase adjustment/addition circuit provided in the wave transmission circuit 12. The reception circuit 13 amplifies the reflection echo signal received by the ultrasonic probe 10 with a predetermined gain. The amplified wave reception signals are respectively inputted to the phase adjustment/addition circuit 14 as independent wave reception signals. The number of wave reception signals corresponds with the number of the transducers.

The phase adjustment/addition circuit 14 inputs the wave reception signal amplified by the reception circuit 13 and controls the phases of the amplified wave reception signals. Then, the phase adjustment/addition circuit 14 forms an ultrasonic beam for a single or a plurality of convergence points. The wave reception signal from the phase adjustment/ addition circuit 14 is inputted to the signal processing section 15. The signal processing section 15 performs signal processing, such as gain correction, logarithmic compression, wave detection, profile enhancement, and filtering.

The ultrasonic probe 10, the ultrasonic wave transmission and reception control circuit 11, the wave transmission circuit 12, the reception circuit 13, the phase adjustment/addition circuit 14, and the signal processing section 15 constitute an ultrasonic wave transmitting and receiving means. A single tomographic image is acquired by an ultrasonic beam being swept within the body of the subject 1 in a fixed direction, using the ultrasonic probe 10.

The black-and-white scan converter 16 includes a tomographic scanning means. The tomographic scanning means acquires, at an ultrasonic wave intervals, ultrasonic wave reception signal frame data representing the interior of the subject 1, including a locomotive tissue, by use of a reflection echo signal outputted from the signal processing section 15 of the ultrasonic wave transmitting and receiving means. The tomographic scanning means reads the ultrasonic wave reception signal frame data at a television-system period to display the ultrasonic wave reception signal frame data. The black-and-white scan converter 16 also includes a means for performing system control. The black-and-white scan converter 16 includes, for example, an analog-to-digital converter, a plurality of frame memories, and a controller. The analog-to-digital converter converts the reflection echo signal outputted from the signal processing section 15 to a digital signal. The frame memories store the tomographic image data, which has been digitalized by the analog-to-digital converter, in time-sequence. The controller controls the operations of the analog-to-digital converter and the frame memories.

The image display unit 18 displays the time-series tomographic image data acquired by the black-and white scan converter 16, or in other words, a B-mode tomographic image. The image display unit 18 includes a digital-to-analog converter and a color monitor. The digital-to-analog converter converts the image data outputted from the black-and-white scan converter 16 via a switching/adding unit 17, to an analog signal. The color monitor receives the analog video signal from the digital-to-analog converter, and displays it as an image.

The ultrasonic wave reception signal frame data selecting section 19 and the displacement measuring section 20 are provided in a circuit path branching out from the output end of the phase adjustment/addition circuit 14. The pressure measuring section 21 is provided in parallel with the ultrasonic wave reception signal frame data selecting section 19 and the displacement measuring section 20. The distortion and elastic modulus calculating unit 23 is provided in the stage following the pressure measuring section 21 and the displacement measuring section 20. The elasticity data processing section 24 and the color scan converter 25 are provided in the stage following the distortion and elastic modulus calculating unit 23. The switching/adding unit 17 is provided on the output side of the black-and-white scan converter 16 and the color scan converter 25. An operator or the like can freely control the color scan converter 25, via a device control interface section (not shown).

The ultrasonic wave reception signal frame data selecting section 19 sequentially stores the ultrasonic wave reception signal frame data in a frame memory provided in the ultrasonic wave reception signal frame data selecting section 19 (the currently stored ultrasonic wave reception signal frame data will be referred to as ultrasonic wave reception signal frame data N). The ultrasonic wave reception signal frame data is sequentially and consecutively outputted from the phase adjustment/addition circuit 14 at a frame rate of the ultrasonic imaging device. In accordance with a control instruction from the ultrasonic imaging device, the ultrasonic wave reception signal frame data selecting section 19 selects one piece of ultrasonic wave reception signal frame data from among the past ultrasonic wave reception signal frame data N-1, N-2, N-3,..., N-M (the selected ultrasonic wave reception signal frame data will be referred to as ultrasonic wave reception signal frame data X). Then, the ultrasonic wave reception signal frame data selecting section 19 outputs the ultrasonic wave reception signal frame data N and the ultrasonic wave reception signal frame data X to the displacement measuring section 20 as one pair. The signal outputted from the phase adjustment/addition circuit 14 is not limited to the ultrasonic wave reception signal frame data. The signal may be an (I, Q) signal obtained through composite demodulation of the ultrasonic wave reception signals.

The ultrasonic wave reception signal frame data selecting section 19 acquires information on the interval between the selected pair of ultrasonic wave reception signal frame data N and X. A pressing action performed by the automatic pressing mechanism 22 is controlled depending on the interval between the selected pair of ultrasonic wave reception signal frame data N and X. An example of the pressing action will be described, hereafter.

The interval between the pair of ultrasonic wave reception signal frame data N and X, selected by the ultrasonic wave reception signal frame data selecting section 19, is determined by the interval of the ultrasonic wave reception signal frame data outputted from the phase adjustment/addition circuit 14 and inputted to the ultrasonic wave reception signal frame data selecting section 19, and the number of ultrasonic wave reception signal frame data between the past ultrasonic wave reception signal frame data X and the current ultrasonic wave reception signal frame data N, composing the pair of ultrasonic wave reception signal frame data. For example, if the ultrasonic wave reception signal frame data outputted from the phase adjustment/addition circuit 14 has a frame rate of 40 frames per second and the number of removed frames between the pair of ultrasonic wave reception signal frame data N and X is 1, the frame rate between the pair of ultrasonic wave reception signal frame data is 20 frames per second. The automatic pressing mechanism 22 acquires the information on the interval between the pair of ultrasonic wave reception signal frame data N and X, and controls the pressing speed of the pressing action based on the acquired interval information.

For example, under the above conditions, when the frame rate of the ultrasonic wave reception signal frame data outputted from the phase adjustment/addition circuit 14 is 40 frames per second, and the frame rate between the pair of ultrasonic wave reception signal frame data N and X is 20 frames per second, it is presumed that pressure is consecutively applied to the tissue of interest at a pressing speed V0. The pressing speed V0 produces a distortion of 0.7% to the tissue of interest, which is suitable for improving the image quality. Under the conditions, when the frame rate of the ultrasonic wave reception signal frame data outputted from the phase adjustment/addition circuit 14 is changed to a frame rate of 20 frames per second due to changes in the imaging conditions of the ultrasonic imaging device, the frame rate between the pair of ultrasonic wave reception signal frame data N and X becomes half or decreases to 10 frames per second. At this time, when the pressure is still being applied at the pressing speed V0, the intermittent period between the ultrasonic wave reception signal frame data doubles in length. Therefore, the distortion applied to the tissue of interest increases to 1.4%, deviating from the range of distortion amount suitable for improving the image quality. As a result, the outputted consecutive elasticity image data forms a deteriorated image.

Therefore, the automatic pressing mechanism 22 acquires the information on the interval of the ultrasonic wave reception signal frame data and halves the pressing speed to V0/2, for example, under the above conditions. As a result, even when the ultrasonic wave transmission and reception cycle changes due to the changes in the imaging conditions of the ultrasonic imaging device, the pressing action can be automatically controlled so that the pressing speed becomes optimal for acquiring a high-quality elasticity image.

In addition, the automatic pressing mechanism 22 can arbitrarily switch the settings of the pressing action, such as the pressing speed, the cumulative compression amount (amplitude) during a continuous pressure increasing and decreasing processes, and the pressure threshold for stopping the pressing action.

The displacement measuring section 20 performs a one-dimensional or a two dimensional correlation process, based on the pair of ultrasonic wave reception signal frame data selected by the ultrasonic wave reception signal frame data selecting section 19. The displacement measuring section 20 measures the displacement or the motion vector (the direction and distance of the displacement) of each measuring point on the tomographic image and generates displacement frame data. As methods used to detect the motion vector, for example, there are the block matching method and the gradient method, such as those described in Patent Document 1. In the block matching method, the images are divided into blocks composed of, for example, NxN pixels. Then, a block that is the most similar to a block of interest in the current frame is searched from the previous frame. Predictive coding is performed with reference to the searched block.

The pressure measuring section 21 measures or estimates the pressure applied to the to-be-imaged portion of the subject 1. The pressure measuring section 21 measures the pressure applied between the probe head of the ultrasonic probe 10 and the subject 1. For example, the pressure measuring section 21 may be configured so that a pressure sensor is attached to the side surface of the probe head. The pressure sensor detects the pressure applied to a stick-shaped member. The pressure measuring section 21 measures the pressure between the probe head and the subject 1 at an arbitrary time and sends the measured pressure value to the distortion and elastic modulus calculating section 23. No particular limitation is imposed on the type of pressure sensor. For example, a capacitive or a resistance wire-type pressure sensor may be used.

The distortion and elastic modulus calculating section 23 calculates the distortion and the elastic modulus at each measuring point on the tomographic image from the displacement frame data (the amount of movement) and the pressure respectively outputted from the displacement measuring section 20 and the pressure measuring section 21. The distortion and elastic modulus calculating section 23 generates numeric data (elasticity frame data) on the distortion and the elastic modulus and outputs the numeric data to the elastic data processing section 24. The calculation of the distortion performed by the distortion and elastic modulus calculating section 23 does not require data on pressure. The distortion may be determined through calculation by spatially differentiating the displacement. In addition, for example, Young's modulus of elasticity Ym, which is one elastic modulus, is determined by dividing the stress (pressure) at each calculation point by the amount of distortion at each calculation point, as shown in the following equation.

$$\mathtt{Ymij \ = \ pressure \ (stress) \ ij/(distortion \ amount \ ij)}$$

$$\mathtt{(i,j=1,2,3…)}$$

Here, the indicators i and j indicate the coordinates of the frame data.

The elasticity data processing section 24 performs various image processing on the elasticity frame data from the distortion and elastic modulus calculating section 23. The image processing includes, for example, a smoothing process within the coordinate plane, a contrast optimization process, and a smoothing process in the time axis direction between frames. Then, the elasticity data processing section 24 outputs the processed elasticity frame data to the color scan converter 25.

The color scan converter 25 includes a color information converting means, which receives the elasticity frame data outputted from the elasticity data processing section 24, and an instruction from a device control interface 216 or an upper limit and a lower limit of a gradation-imparting selection range for the elasticity frame data outputted from the elasticity data processing section 24, and provides, as elasticity image data, information of colors, such as red, green, and blue, determined from the elasticity frame data. For example, with regards to a region in the elasticity frame data outputted from the elasticity data processing section 24 in which a large amount of distortion is measured, the color information converting means changes the corresponding region within the elasticity image data to a red color code. On the other hand, with regards to a region in which a small amount of distortion is measured, the color information converting means changes the corresponding region within the elasticity image data to a blue color code. The color scan converter 25 may be a black-and-white converter. In this case, with regards to the region in which a large amount of distortion is measured, the brightness of the corresponding region within the elasticity image data is increased, and with regards to the region in which a small amount of distortion is measured, the brightness of the corresponding region within the elasticity image data is decreased.

The black-and-white tomographic image data from the black-and-white scan converter 16 and the colored elasticity image data from the color scan converter 25 are inputted to the switching/adding unit 17. The switching/adding unit 17 switches or adds the images, so as to output only the black-and-white tomographic image data or the colored elasticity image data, or output both the image data after adding them for composition. In addition, for example, as described in Patent Document 2, the black-and-white tomographic image, and the colored elasticity image or the black-and-white elasticity image from the black-and-white scan converter may be simultaneously displayed on a two-screen display. Furthermore, for example, the colored elasticity image may be made translucent and superposed on the black-and-white tomographic image. The image data outputted from the switching/adding unit 17 is outputted to the image display unit 18.

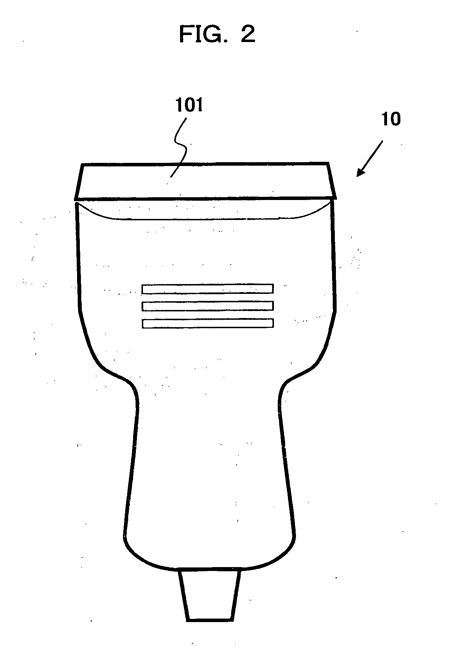

FIG. 2 is an outside view of a generally-used, one-dimensional linear array ultrasonic probe. A group of transducer elements is aligned and disposed on an ultrasonic transmission and reception surface 101 of the ultrasonic probe 10. The group of transducer elements serves as a source of ultrasonic waves, and also receives reflection echo signals. Each transducer generally functions to convert an input wave transmission signal in the form of a pulse wave or a continuous wave to ultrasonic waves, and radiate the ultrasonic waves. The transducers also function to receive the ultrasonic waves reflected from the interior of the subject 1, convert the reflected ultrasonic wave into an electrical reception wave signal, and output the reception wave signal.

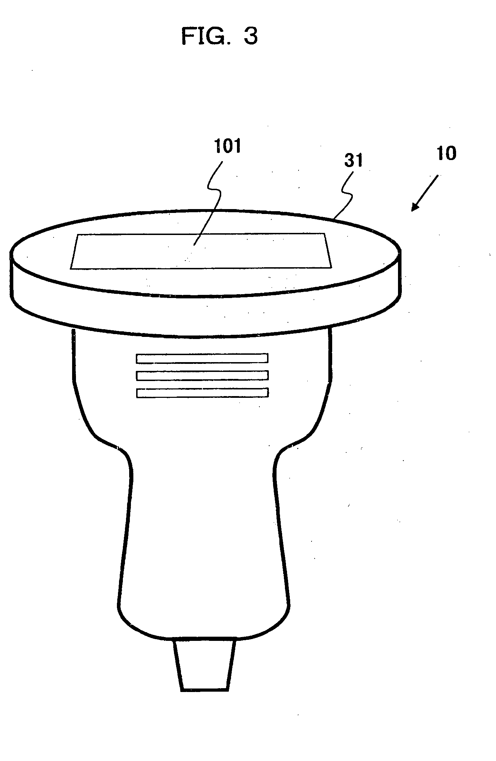

FIG. 3 is an outside view of the ultrasonic probe 10 for acquiring an elasticity image using ultrasonic waves. The ultrasonic probe 10 includes a pressing plate 31 attached such that the surface of the pressing plate becomes flush with the ultrasonic wave transmitting and receiving surface 101. The center of the pressing plate 31 is cut out so that the ultrasonic wave transmitting and receiving surface 101 is exposed. When acquiring the elasticity image, the ultrasonic waves are transmitted and received via the ultrasonic wave transmitting and receiving surface 101. At the same time, in order to apply a distributed stress to the to-be-imaged portion of the subject 1, a pressing surface formed by the ultrasonic wave transmitting and receiving surface 101 and the pressing plate 31 is brought into contact with the subject 1, and is moved vertically so as to press the subject 1. The vertical movement of the pressing surface may be performed manually by an operator. However, the vertical movement may be performed by the automatic pressing mechanism 22 such as those described below.

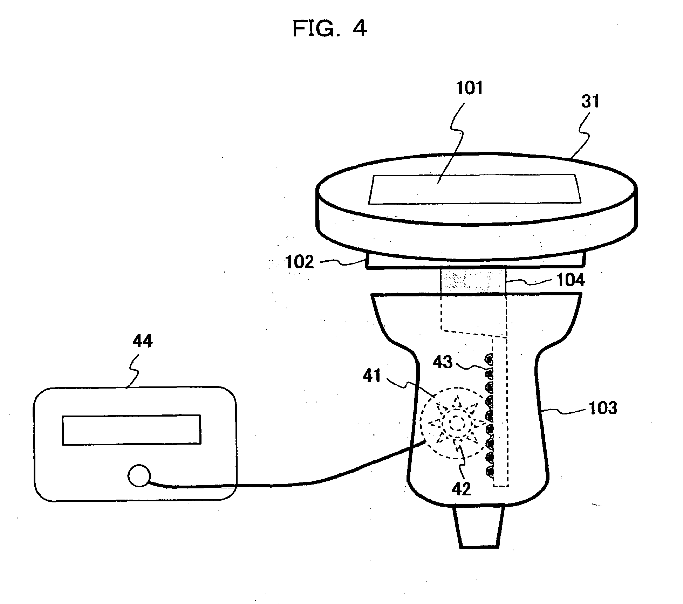

FIG. 4 is a diagram showing an illustrative example of the automatic pressing mechanism 22 that performs pressing action of the ultrasonic probe, in which a driving force from an actuator including a motor mechanism is used. In FIG. 4, the automatic pressing mechanism 22 vertically moves a pressing stage 102, which has an independent pressing surface formed by the ultrasonic wave transmitting and receiving surface 101 and the pressing plate 31. The automatic pressing mechanism 22 includes a rack and pinion composed of a pinion 42 and a rack 43. The pinion 42 is provided on a rotation shaft of a motor mechanism 41 held in a probe gripping section 103 of the ultrasonic probe 10. The probe gripping section 103 is gripped by the operator. The rack 43 is provided on a supporting member 104 of the pressing stage 102. The motor mechanism 41 moves the pressing stage 102 vertically in relation to the probe gripping section 103, via the rack and pinion, in accordance with control instructions from an external motor control section 44. In other words, when the operator grips the probe gripping section 103 and places the pressing stage 102 in contact with the subject 1, pressure is applied to the subject 1, via the pressing stage 102, by the actuator changing the distance between the pressing stage 102 and the probe gripping section 103.

A switch (not shown) is disposed in a position that enables the operator to manipulate the switch by the fingers on the hand gripping the probe gripping section 103. The switch is an interface allowing the operator to operate the automatic pressing mechanism 22 (motor control section 44). Through manipulation of the switch, the operator can turn the automatic pressing mechanism 22 on and off, and adjust the operation pressure, the operation cycle, etc. The interface for operating the automatic pressing mechanism 22 is not limited to the above-described switch that is manipulated by the fingers on the hand. The interface may also be, for example, a foot switch that can be operated by the foot.

The motor mechanism 41 may include a mechanism using an electromagnetic motor, an ultrasonic motor, or the like. The power transmission mechanism transmitting power from the motor mechanism 41 to the pressing stage 102 is not limited to the rack and pinion. For example, a cam may be provided in the motor mechanism 41, and the supporting member 104 may be driven in the vertical direction in accordance with the shape of the cam. In addition, a direct motor or the like may be connected directly to the pressing stage 102, thereby driving the pressing stage 102 without the use of a power transmission mechanism such as the rack and pinion.

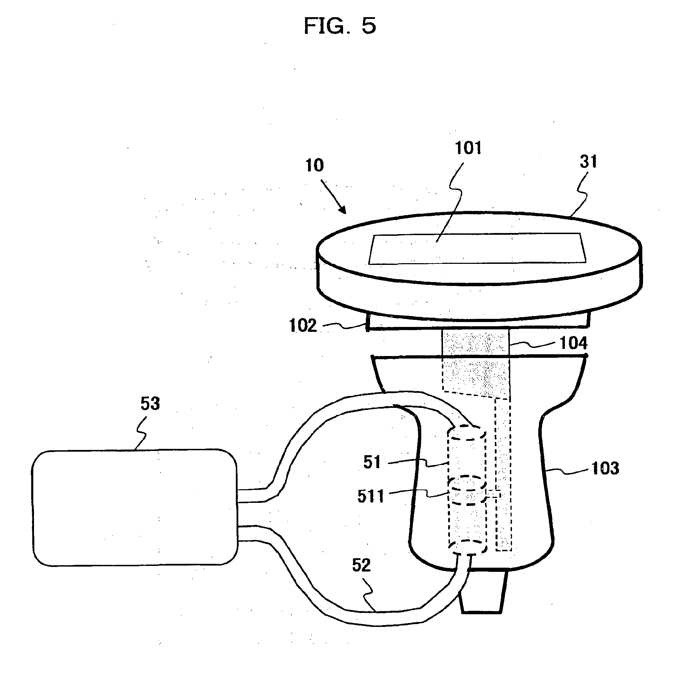

FIG. 5 is a diagram showing another illustrative example of the automatic pressing mechanism 22, in which the driving force from a pump mechanism is used. In FIG. 5, the automatic pressing mechanism 22 includes a reciprocating cylinder 51 held in the probe gripping section 103 of the ultrasonic probe 10. The probe gripping section 103 is gripped by the operator. The supporting member 104 of the pressing stage 102 is connected to a piston 511 of the cylinder 51. The cylinder 51 is connected to an inlet port and an outlet port of a pump 53 by a tube 52. The piston 511 provided within the cylinder 51 is moved vertically under the pressure control of the pump 53. With the structure in which the pressing stage 102 is moved together with the piston, the pressing stage 102 is automatically moved in the vertical direction. Similarly, a switch (not shown) is disposed in a position which enables the operator to manipulate the switch by the fingers on the hand gripping the probe gripping section 103. The switch is an interface allowing the operator to operate the automatic pressing mechanism 22 (pump 53). No particular limitation is imposed on the working fluid of the pump mechanism, and the working fluid may be water, oil, air, etc.

Examples in which driving mechanisms for driving the pressing stage 102, such as the motor mechanism and the pump mechanism, are provided on the probe gripping section 103 side have been shown. However, the driving mechanism may be provided on the pressing stage 102 side. In addition, examples in which the automatic pressing mechanism 22 is installed within the ultrasonic probe 10 are described. However, the automatic pressing mechanism 22 may be externally attached to an existing ultrasonic probe.

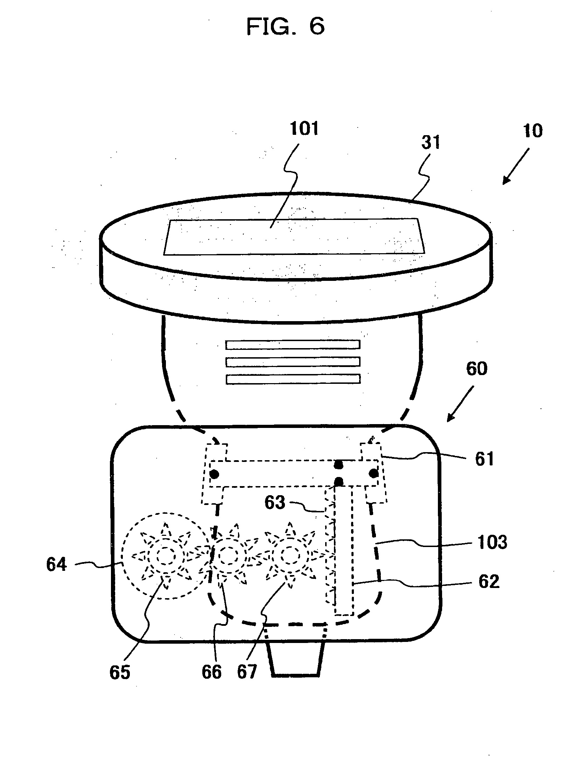

FIG. 6 is a diagram showing another illustrative example of the automatic pressing mechanism 22, in which an automatic pressing mechanism 60 is externally attached to an existing ultrasonic probe, so that the automatic pressing unit 60 can perform the operation equivalent to that of the drive of the pressing stage. The automatic pressing unit 60 includes an ultrasonic probe fixing mechanism 61 and a driving mechanism 62. The ultrasonic probe fixing mechanism 61 fixedly holds the existing ultrasonic probe 10. The driving mechanism 62 drives the ultrasonic probe fixing mechanism 61 in a straight direction (vertical direction). Similarly, a switch (not shown) is disposed in a position which allows the operator to manipulate the switch by the fingers on the hand gripping the automatic pressing unit 60. The switch is an interface allowing the operator to operate the automatic pressing unit 60. The ultrasonic probe fixing mechanism 61 comes into pressure contact with a neck portion of the probe gripping section 103 of the ultrasonic probe 10, so as to fixedly hold the ultrasonic probe 10. The ultrasonic probe 10 that is fixed by such an ultrasonic probe fixing mechanism 61 is equivalent to the pressing stage, such as that shown in FIG. 4. The probe gripping section 103, namely the ultrasonic probe 10, is moved vertically, using a rack and pinion composed of a rack 63 and a pinion 65. The rack 63 is provided on a supporting member 62 of the ultrasonic probe fixing mechanism 61. The pinion 65 is provided on the rotation axis of the driving mechanism (motor mechanism) 64. In FIG. 6, two gears 66 and 67 for power transmission are provided between the rack 63 and the pinion 65. A casing that includes such an automatic pressing unit 60 is removably attached to the outside of the casing of the existing ultrasonic probe 10. When the operator grips the automatic pressing unit 60, the ultrasonic probe 10 itself can be moved vertically as the pressing stage.

Next, there will be described an example in which the pressure measuring section 21 measures the pressure applied to the skin of the subject 1 from the pressing surface, and the operation of the automatic pressing mechanism 22 is controlled using the pressure data.

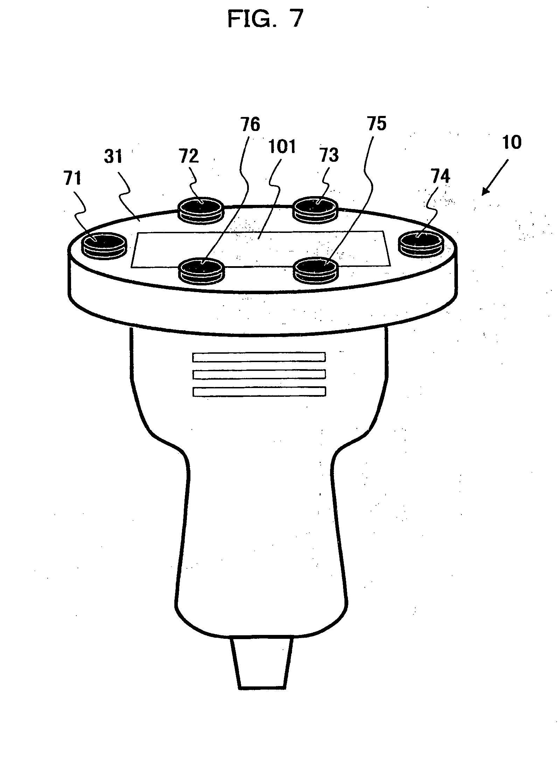

FIG. 7 is a diagram showing an example of the ultrasonic probe 10 including a pressure measuring section 21 for measuring the pressure applied between the ultrasonic wave transmitting and receiving surface 101 of the ultrasonic probe 10 and the skin of the subject 1. As shown in FIG. 7, the ultrasonic probe 10 includes the pressure measuring section 21 including pressure sensors 71 to 76. The pressure sensors 71 to 76 are disposed on the peripheral edge portion of the pressing plate 31. Using such an ultrasonic probe 10, as shown in FIG. 1, the pressure measuring section 21 measures the pressure between the pressing plate 31 and the skin of the subject 1 at an arbitrary time and outputs the pressure data to the automatic pressing mechanism 22 and the distortion and elastic modulus calculating section 23. In other words, the automatic pressing mechanism 22 acquires the pressure data measured by the pressure measuring section 21 and controls the pressing action of the automatic pressing mechanism 22 based on the pressure data. The pressure measuring section 21 may acquire pressure data by measuring the load acting on the driving mechanism of the automatic pressing mechanism 22 and calculating the pressure applied to the skin of the subject 1 from the pressing surface based on the measured load.

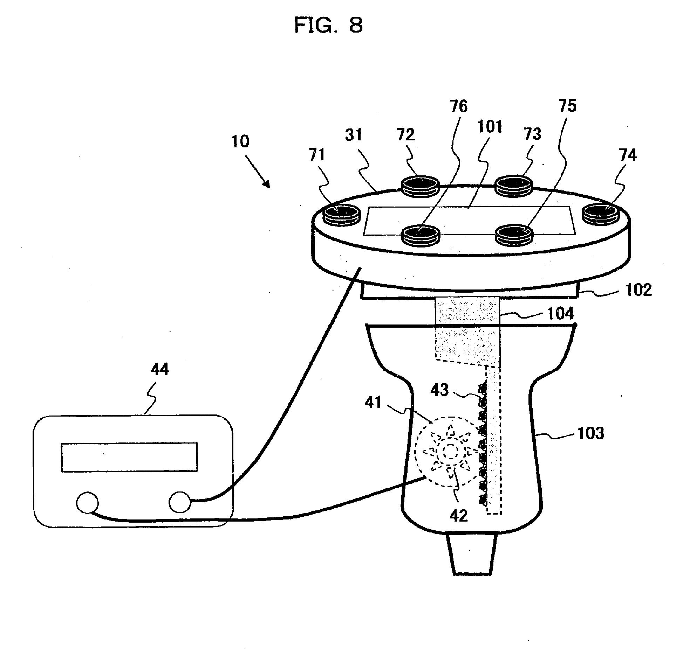

There will be described a case where, as shown in FIG. 1, the automatic pressing mechanism 22 and the pressure measuring section 21 are connected together in order to control the operation of the automatic pressing mechanism 22. FIG. 8 is a diagram of an example of the automatic pressing mechanism 22 in which the driving power generated by the motor mechanism 41 shown in FIG. 4 is used. As shown in FIG. 8, the pressure data from the pressure sensors 71 to 76 attached to the peripheral edge portion of the pressing plate 31 is inputted to the motor control section 44 of the automatic pressing mechanism 22. The motor control section 44 outputs a motor control signal corresponding to the pressure data to the motor mechanism 41, and controls the motor mechanism 41 so that the motor mechanism 41 performs the desired pressing action.

Through the use of the automatic pressing mechanism 22, the operation of the automatic pressing mechanism 22 can be stopped when the pressure measuring section 21 measures a pressure higher than a certain reference level. As a result, excessive pressure is not applied to the subject. In addition, when capturing an elasticity image, there is a pressure range within which a high-quality elasticity image can be obtained. When pressure exceeding the upper limit or pressure below the lower limit is applied, it is known that the elasticity image deteriorates.

In the automatic pressing mechanism 22, when the pressure measuring section 21 measures a pressure higher than a certain threshold during a certain continuous pressure increasing process, the operation of the automatic pressing mechanism 22 can be controlled so that the pressure increasing process is switched to the continuous pressure decreasing process. On the other hand, when the pressure measuring section 21 measures a pressure lower than a certain threshold during a certain continuous pressure decreasing process, the operation of the automatic pressing mechanism 22 can be controlled so that the pressure decreasing process is switched to the continuous pressure increasing process. Through the repetition of this operation, an appropriately pressed state can be always maintained. As a result, a high-quality elasticity image can be efficiently acquired within a limited imaging time.

Next, there will be described an intra-corporeal ultrasonic probe according to further illustrative examples and embodiments of the present invention used for acquiring an elasticity image of the subject 1 by use of ultrasonic waves. The ultrasonic probe may assume a different shape in accordance with a portion of the subject into which the ultrasonic probe is inserted. That is, there exist ultrasonic probes of various types, such as a per-oral-type, a per-anal-type, a transvaginal-type, or an intravascular-type. The present invention can be applied regardless of the shape or type of ultrasonic probe. Hereafter, a transrectal probe that is inserted into the rectum via the anus of the subject will be described as an example.

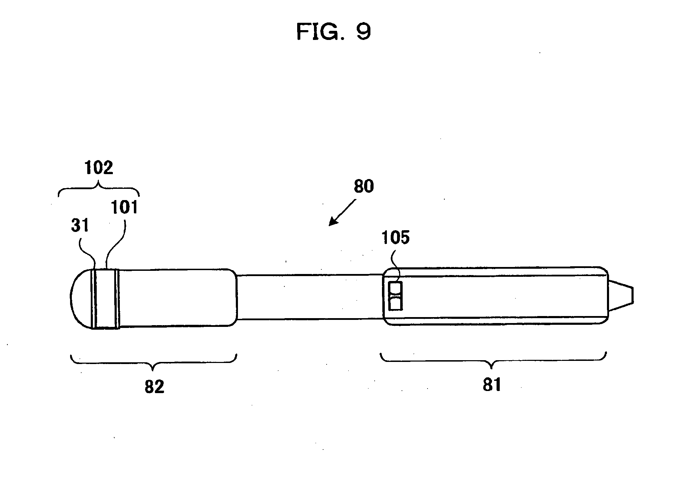

FIG. 9 is an outside view of a transrectal ultrasonic probe 80 according to an illustrative example useful to understand the present invention. The transrectal ultrasonic probe 80 is equivalent to the ultrasonic probe 10 in FIG. 1. When the operator grips a probe gripping section 81 and inserts an intra-corporeal section 82 into the rectum of the subject 1, the ultrasonic wave transmitting and receiving surface 101 comes into contact with the inner surface of the rectum of the subject. The pressing stage 102 including the pressing surface formed by the ultrasonic wave transmitting and receiving surface 101 and the pressing plate 31 can be moved relative to the intra-corporeal section 82. Then, the pressing stage 102 is pressed against the inner surface of the rectum of the subject 1 by the automatic pressing mechanism 22 incorporated in the intra-corporeal section 82. A switch 105 is disposed in a position which allows the operator to manipulate the switch by the fingers on the hand gripping the probe gripping section 81. The switch is an interface allowing the operator to operate the automatic pressing mechanism 22.

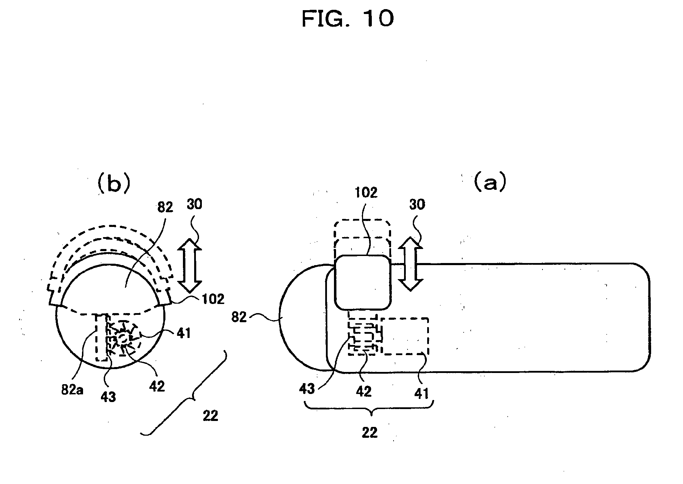

FIG. 10(a) is a partially enlarged view showing an illustrative example of the automatic pressing mechanism 22 provided in the transrectal ultrasonic probe 80. FIG. 10(b) is a diagram of the ultrasonic probe 80 in FIG. 10(a) viewed from the left-hand side. The automatic pressing mechanism 22 moves the pressing stage 102 relative to the intra-corporeal section 82, in the direction indicated by the vertical arrow 30 in the drawings. The automatic pressing mechanism 22 moves the pressing stage 102 by the action of the actuator including the motor mechanism 41, the pinion 42, and the rack 43. The automatic pressing mechanism 22 includes the motor mechanism 41 and the rack and pinion. The motor mechanism 41 is installed and held within the intra-corporeal section 82. The rack and pinion includes the pinion 42 and the rack 43. The pinion 42 is provided on the rotation shaft of the motor mechanism 41. The rack 43 is provided on a supporting member 87 of the pressing stage 102.

The motor mechanism 41 is controlled by the switch 105 provided on the probe gripping section 81. The motor mechanism 41 moves the pressing stage 102 vertically, via the rack and pinion. In other words, the operator can turn the automatic pressing mechanism 22 on and off, and adjust the operation pressure, the operation cycle, etc., via the switch. When the operator grips the probe gripping section 81 and inserts the intra-corporeal section 82, including the pressing stage 102, into the subject 1, the actuator changes the distance between the pressing stage 102 and the surface of the intra-corporeal section 82. Therefore, pressure can be applied to the subject 1, via the pressing stage 102. In other words, a surface of the intra-corporeal section 82 opposite the pressing stage 102 serves as a supporting surface, and comes into contact with a surface portion of the inner surface of the rectum of the subject 1, the surface potion being opposite to the portion to be imaged. Therefore, when the actuator changes the distance between the pressing stage 102 and the supporting surface, pressure is applied to the inner surface of the rectum of the subject with which the pressing stage 102 is in contact.

The motor mechanism 41 may include a mechanism using an electromagnetic motor, an ultrasonic motor, or the like. The power transmission mechanism transmitting power from the motor mechanism 41 to the pressing stage 102 is not limited to the rack and pinion. For example, a cam may be provided in the motor mechanism 41, and the supporting member 104 may be driven in the vertical direction in accordance with the shape of the cam. In addition, a direct motor or the like may be connected directly with the pressing stage 102, thereby driving the pressing stage 102 without the use of a power transmission mechanism, such as the rack and pinion.

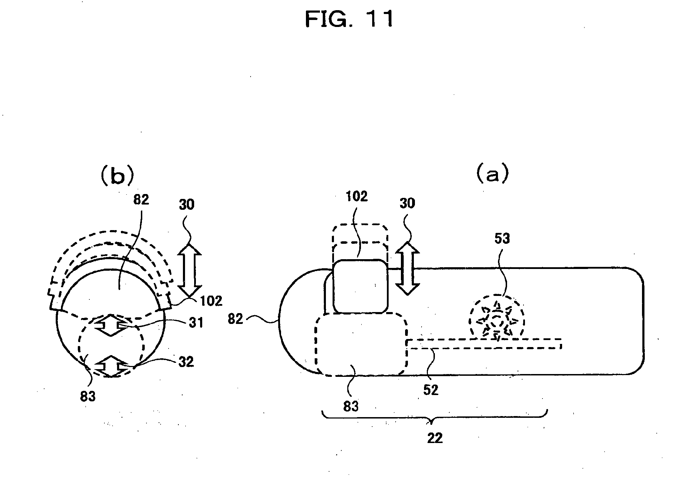

FIG. 11(a) is a partially enlarged view showing another illustrative example of the automatic pressing mechanism 22 provided in the transrectal ultrasonic probe 80. FIG. 11(b) is a diagram of the ultrasonic probe 80 in FIG. 11(a) viewed from the left-hand side. Fluid is supplied to and discharged from a pressing bag 83 by a pump 53 and a tube 52, thereby inflating and deflating the pressing bag 83 in the directions of the bidirectional arrows 31 and 32. As a result, the pressing stage 102 moves relative to the intra-corporeal section 82, along the direction of the vertical arrow 30 in the drawing, whereby pressure is applied to the inner surface of the rectum of the subject 1 with which the pressure stage 102 is in contact. In this case as well, a switch (not shown) is disposed in a position which allows the operator to manipulate the switch by the fingers on the hand gripping the probe gripping section 81. The switch is an interface allowing the operator to operate the automatic pressing mechanism 22 (pump 53). No particular limitation is imposed on the working fluid of the pump mechanism, and the working fluid may be water, oil, air, etc.

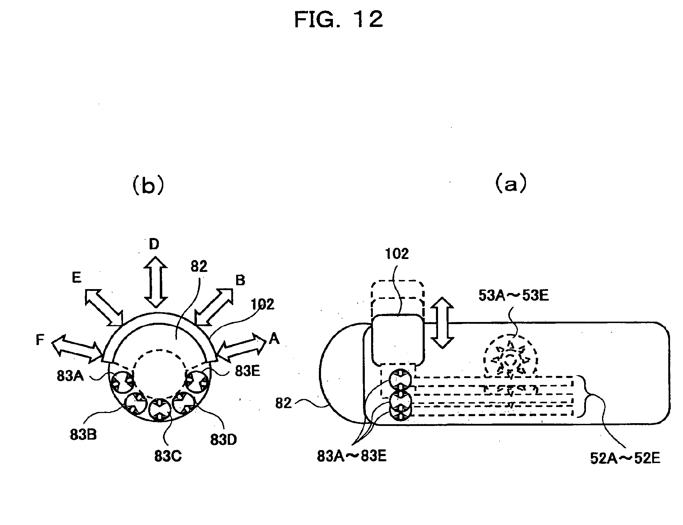

FIG. 12(a) is a partially enlarged view showing another illustrative example of the automatic pressing mechanism 22 provided in the transrectal ultrasonic probe 80. FIG. 12(b) is a diagram of the ultrasonic probe 80 in FIG. 12(a) viewed from the left-hand side. The automatic pressing mechanism 22 is configured so that five systems including the pump, tube, and pressing bag are provided on the opposite side of the pressing stage 102. The pump, tube, and pressing bag are the same as those used in the example described in FIG. 11(a) and Fig. 11(b). The pressing bags 83A, 83B, 83C, 83D, and 83E are respectively inflated and deflated by pumps 53A, 53B, 53C, 53D, and 53E, and tubes 52A, 52B, 52C, 52D, and 52E. By the pressing bags 83A, 83B, 83C, 83D, and 83E being selectively inflated and deflated, the pressing stage 102 can be moved relative to the intra-corporeal section 82, in the respective directions of arrows A, B, C, D, and E, shown in FIG. 12(b). As a result, pressure may be applied to the inner surface of the rectum of the subject 1 in the direction desired by the operator.

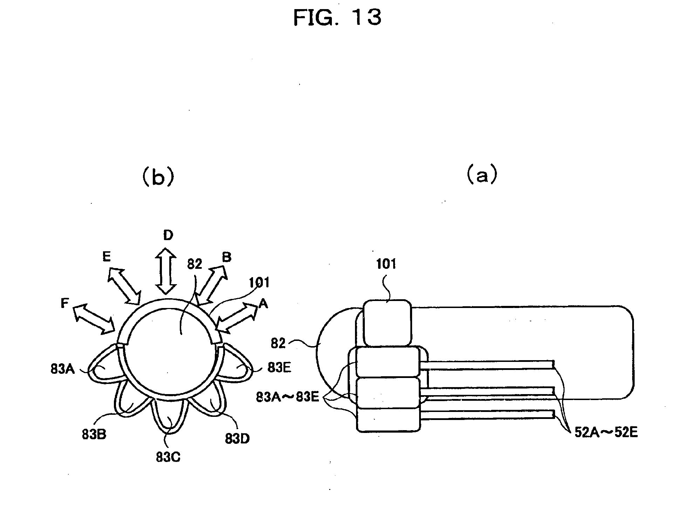

FIG. 13(a) is a partially enlarged view showing another illustrative example of the automatic pressing mechanism 22 provided in the transrectal ultrasonic probe. FIG. 13(b) is a diagram of the ultrasonic probe in FIG. 13(a) viewed from the left-hand side. The automatic pressing mechanism 22 is configured so that the pressing bags 83A, 83B, 83C, 83D, and 83E are attached to the outside of the existing transrectal ultrasonic probe 80. The pressing bags 83A, 83B, 83C, 83D, and 83E are the same as those used in the example described in FIG. 12(a) and FIG. 12(b). The pressing bags 83A, 83B, 83C, 83D, and 83E are respectively inflated and deflated by five pumps (not shown) connected via tubes 52A, 52B, 52C, 52D, and 52E. The surface of the pressing bags 83A, 83B, 83C, 83D, and 83E serves as a supporting surface, which comes into contacts with a surface area of the inner surface of the rectum of the subject 1, the surface area being opposite to a portion to be imaged. By the pressing bags 83A, 83B, 83C, 83D, and 83E being selectively inflated and deflated, the entire intra-corporeal section 82 can be moved relative to the rectum of the subject 1, in the respective directions of arrows A, B, C, D, and E, shown in FIG. 13(b). As a result, pressure can be applied to the inner surface of the rectum of the subject in the direction desired by the operator, even when the transrectal ultrasonic probe does not have a movable pressing stage.

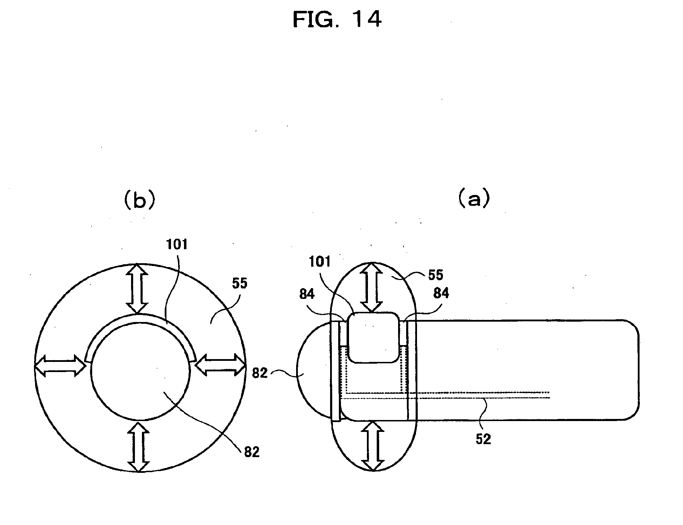

FIG. 14(a) is a partially enlarged view showing another illustrative example of the automatic pressing mechanism 22 provided in the transrectal ultrasonic probe. FIG. 14(b) is a diagram of the ultrasonic probe in FIG. 14(a) viewed from the left-hand side. The automatic pressing mechanism 22 is configured so that a ring-shaped pressing bag 55 is attached to the outside of the existing transrectal ultrasonic probe 80. A pump (not shown), connected to the pressing bag 55 via an opening 84 and the tube 52, supplies liquid (for example, water or saline solution) to the pressing bag 55 and discharges liquid from the pressing bag 55, thereby inflating and deflating the pressing bag 55. The pressing bag 55 is in contact with the inner surface of the rectum of the subject 1. Therefore, by the pressing bag 55 being inflated and deflated, pressure can be applied to the inner surface of the rectum of the subject without moving the ultrasonic wave transmitting and receiving surface 101 relative to the inner surface of the rectum of the subject. The pressing bag 55 is present between the ultrasonic wave transmitting and receiving surface 101 and the inner surface of the rectum of the subject. However, since the pressing bag 55 is filled with liquid, without hindering the transmission and reception of ultrasonic waves, a surface area of the pressing bag 55 in contact with an area of the inner surface of the rectum of the subject where the portion to be imaged is present functions as an ultrasonic wave transmitting and receiving surface. In addition, a surface area of the pressing bag 55 which comes into contacts with a surface area of the inner surface of the rectum of the subject, the surface area being opposite to a portion to be imaged, serves as a supporting surface.

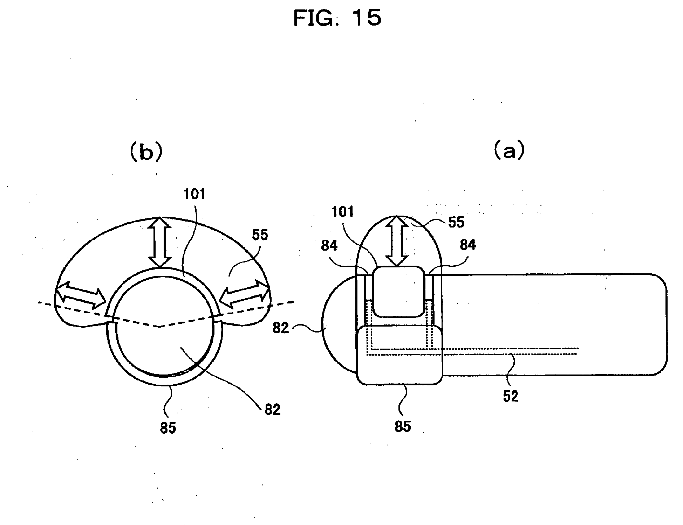

FIG. 15(a) is a partially enlarged view showing another illustrative example of the automatic pressing mechanism 22 provided in the transrectal ultrasonic probe. FIG. 15(b) is a diagram of the ultrasonic probe in FIG. 15(a) viewed from the left-hand side. A stopper 85 is attached to the outside of the ring-shaped pressing bag 55 described in FIG. 14(a) and FIG. 14(b). The stopper 85 restricts the inflating direction of the pressing bag 55. As a result, the inflating direction of the pressing bag 55 is restricted to one direction. Therefore, pressure can be efficiently applied to the inner surface of the rectum of the subject 1. At this time, the surface of the stopper 85 serves as a supporting surface which comes into contacts with a surface area of the inner surface of the rectum of the subject, the surface area being opposite to a portion to be imaged.

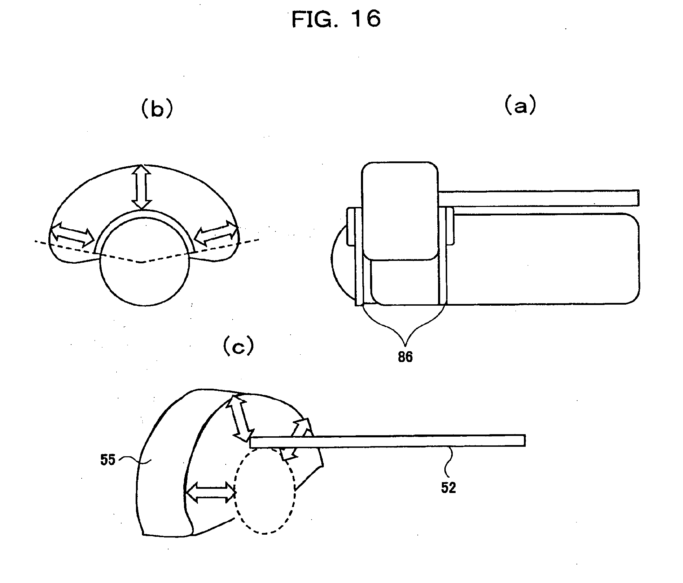

FIG. 16(a) is a partially enlarged view showing another illustrative example of the automatic pressing mechanism 22 provided in the transrectal ultrasonic probe. FIG. 16(b) is a diagram of the ultrasonic probe in FIG. 16(a) viewed from the left-hand side. FIG. 16(c) is a perspective view of only the pressing bag 55 and the tube 52. The automatic pressing mechanism 22 is configured so that the pressing bag 55 is attached to the outside of the existing transrectal ultrasonic probe by use of fixing belts 86. A pump (not shown), connected to the pressing bag 55 via the tube 52, supplies liquid (for example, water or saline solution) to the pressing bag 55 and discharges liquid from the pressing bag 55, thereby inflating and deflating the pressing bag 55. The circumference of the pressing bag 55 is in contact with the inner surface of the rectum of the subject. Therefore, pressure can be applied directly to the inner surface of the rectum of the subject by the pressing bag 55 being inflated and deflated. As a result, the automatic pressing mechanism 22 can be easily attached to the transrectal ultrasonic probe 80 that does not have the opening 84 or the internal tube 52, as does FIG. 14. Although an example in which the pressing bag 55 and the tube 52 are attached by the fixing belts 86 is described, the pressing bag 55 and the tube 52 may be attached using other methods.

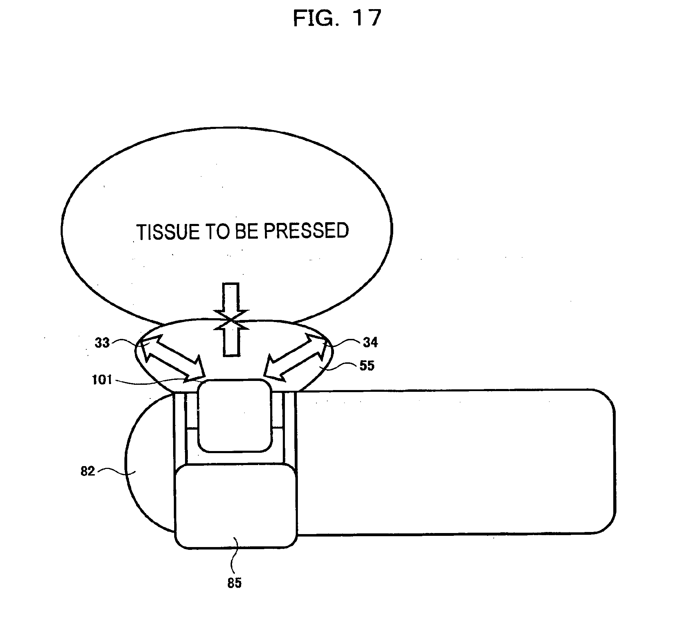

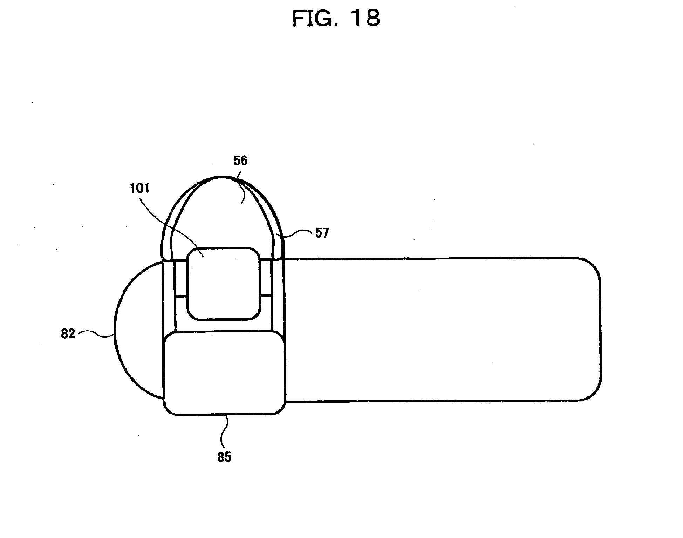

FIG. 17 is a diagram showing another example of the operation of the pressing bag 55 described in FIG. 16 (a). FIG. 18 is a diagram showing another example of the pressing bag. Depending on the shape and the elasticity of the tissue to which pressure is applied by the pressing bag 55, the pressing bag 55 becomes deformed and spreads in the side directions, as indicated by the arrows 33 and 34 in FIG. 17, so that the pressing bag 55 may fail to efficiently press the tissue. Therefore, as shown in FIG. 18, a shell section 57 that restricts the inflating direction of a pressing bag 56 is provided. The shell section 57 requires a lower flexibility than other sections of the pressing bag 56. For example, as a first method, the shell section 57 is made thicker than the other sections of the pressing bag 56. As a second method, a net or the like is adhered to the section of the pressing bag 56 that corresponds to the shell section 57. As a third method, the shell section 57 is formed from a different material having a lower stretchability than the remaining portion of the pressing bag 56. By the shell section 57 being formed using these methods, the pressing bag 55 deforms in predetermined directions, and the pressure may be efficiently applied to the tissue to be pressed.

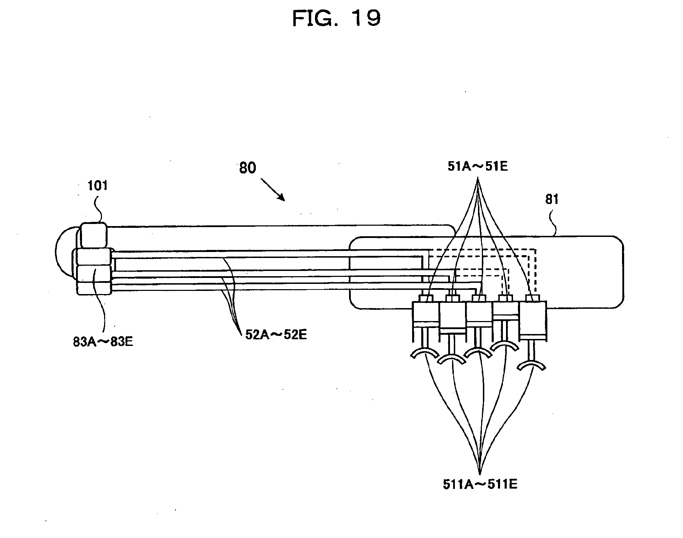

FIG. 19 is a diagram showing a modification of the driving section described in FIG. 13 according to an embodiment of the present invention. The driving section inflates and deflates the pressing bag. In FIG. 13, a configuration is described in which the pressing bags 83A, 83B, 83C, 83D, and 83E, attached to the outside of the existing transrectal ultrasonic probe 80, are respectively inflated and deflated by five pumps (not shown), connected to the bags via the tubes 52A, 52B, 52C, 52D, and 52E. However, in FIG. 19, a driving section including cylinders 51A to 51E and pistons 511A to 511E is provided. The cylinders 51A to 51E are held in the probe gripping section 81. The pistons 511A to 511E move vertically within the cylinders 51A to 51E. The pistons 511A to 511E of the driving section can be operated by fingers (manually). The cylinders 51A to 51E are connected to the pressing bags 83A, 83B, 83C, 83D, and 83E, via the tubes 52A to 25E. Therefore, by the operator arbitrarily operating the pistons 511A to 511E using the fingers on the hand, the working fluid within the cylinders 51A to 51E can be fed to the pressing bags 83A, 83B, 83C, 83D, and 83E. As a result, delicate pressing actions can be performed. In addition, when excessive pressure is applied to the pressing bags 83A, 83B, 83C, 83D, and 83E, the force is transmitted to the fingers. Therefore, pressing conditions such as excessive pressing can be recognized by the fingers.

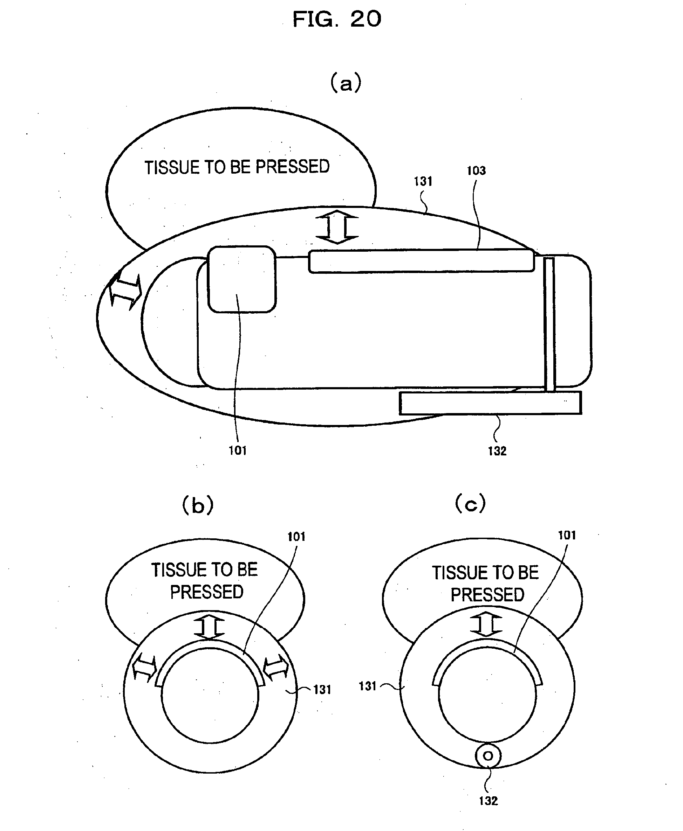

FIG. 20 is a diagram showing a modification of the automatic pressing mechanism 22 described in FIG. 14. The automatic pressing mechanism 22 is provided in the transrectal ultrasonic probe. FIG. 20(a) is a partially enlarged view. FIG. 20(b) is a diagram of the ultrasonic probe in FIG. 20(a) viewed from the left-hand side. FIG. 20(c) is a diagram of the ultrasonic probe in FIG. 20(a) viewed from the right-hand side. The ultrasonic probe includes two probe heads; i.e., the ultrasonic wave transmitting and receiving surfaces 101 and 103. The probe head 101 is a convex-type. The probe head 103 is a linear-type. A pressing bag 131 is provided so as to cover the two ultrasonic wave transmitting and receiving surfaces 101 and 103. By liquid being fed to and removed from the pressing bag 131, the pressing bag 131 is inflated and deflated, thereby applying pressure to the tissue to be pressed. The above-described pump may be used as a power source used to feed the liquid into the pressing bag 131 and remove the liquid from the pressing bag 131, via a tube 132. Alternatively, the liquid may be manually fed to and removed from the pressing bag 131, using a syringe and the like. Since the pressing bag 131 is in contact with the inner surface of the rectum of the subject 1, by the pressing bag 131 being inflated and deflated, pressure can be applied to the inner surface of the rectum of the subject without the ultrasonic wave transmitting and receiving surfaces 101 and 103 being moved relative to the inner surface of the rectum of the subject. The pressing bag 131 is present between the ultrasonic wave transmitting and receiving surfaces 101 and 103 and the inner surface of the rectum of the subject 1. However, since the pressing bag 131 is filled with liquid, without hindering the transmission and reception of ultrasonic waves, a surface area of the pressing bag 131 in contact with an area of the inner surface of the rectum of the subject where the portion to be imaged is present functions as an ultrasonic wave transmitting and receiving surface. In addition, a surface area of the pressing bag 131 which comes into contacts with a surface area of the inner surface of the rectum of the subject, the surface area being opposite to a portion to be imaged, serves as a supporting surface.

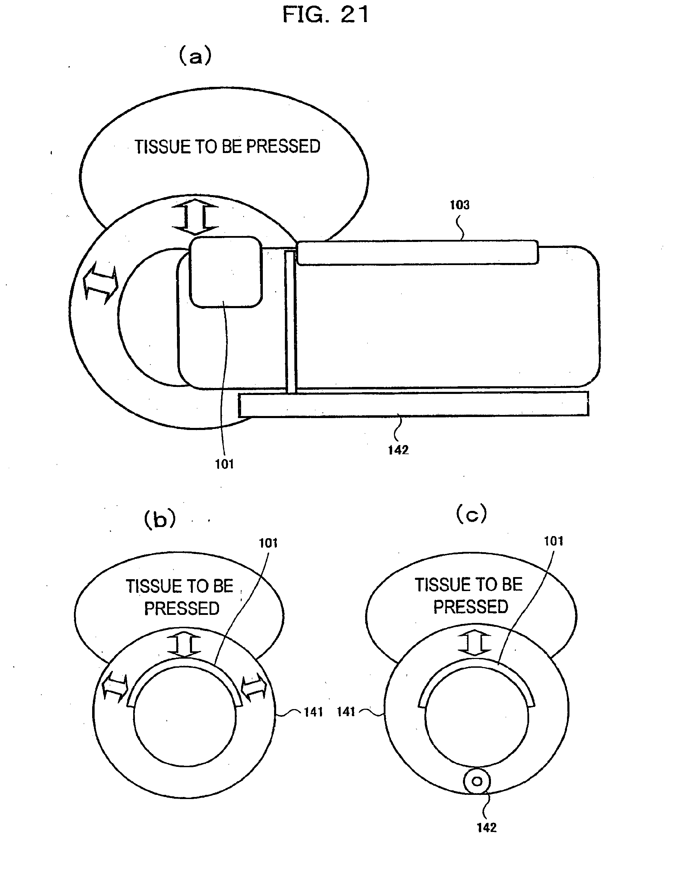

FIG. 21 is a diagram showing a modification of the automatic pressing mechanism 22 described in FIG. 20. The automatic pressing mechanism 22 is provided in the transrectal ultrasonic probe. FIG. 21(a) is a partially enlarged view. FIG. 21(b) is a diagram of the ultrasonic probe in FIG. 21(a) viewed from the left-hand side. FIG. 21(c) is a diagram of the ultrasonic probe in FIG. 21(a) viewed from the right-hand side. The ultrasonic probe includes two probe heads; i.e., the ultrasonic wave transmitting and receiving surfaces 101 and 103. The probe head 101 is a convex-type. The probe head 103 is a linear-type. A pressing bag 141 is provided so as to cover the entire tip of the probe head, including the ultrasonic wave transmitting and receiving surface 101 that serves as a probe head section. The pressing bag 141 is inflated and deflated by liquid being fed to and removed from the pressing bag 141, thereby applying pressure to the tissue to be pressed. The above-described pump may be used as a power source used to feed the liquid into the pressing bag 141 and remove the liquid from the pressing bag 141, via a tube 142. Alternatively, the liquid may be manually fed to and removed from the pressing bag 141, using a syringe and the like.

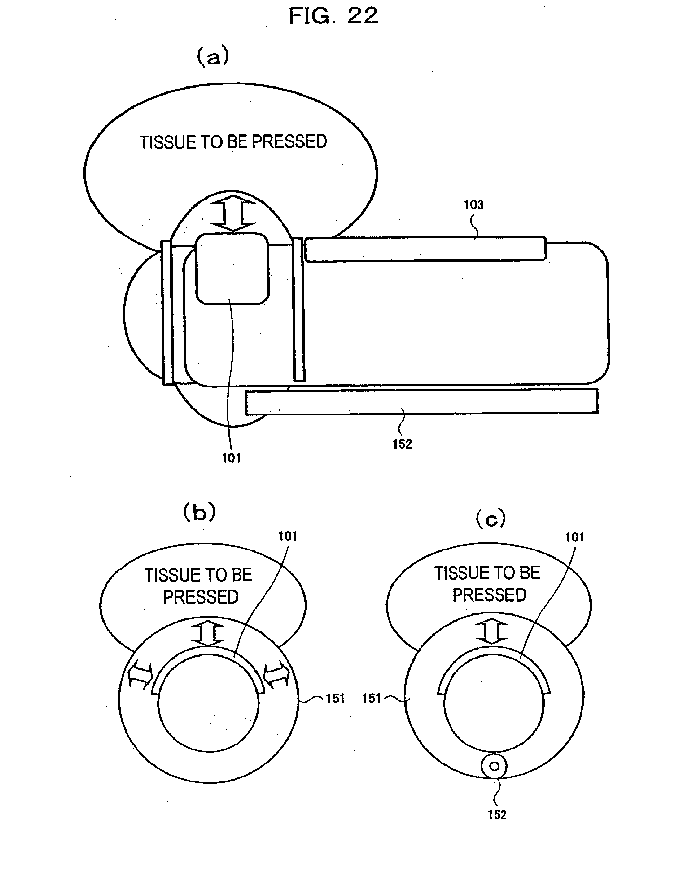

FIG. 22 is a diagram showing a modification of the automatic pressing mechanism 22 described in FIG. 21. The automatic pressing mechanism 22 is provided in the transrectal ultrasonic probe. FIG. 22(a) is a partially enlarged view. FIG. 22(b) is a diagram of the ultrasonic probe in FIG. 22(a) viewed from the left-hand side. FIG. 22(c) is a diagram of the ultrasonic probe in FIG. 22(a) viewed from the right-hand side. The ultrasonic probe includes two probe heads; i.e., the ultrasonic wave transmitting and receiving surfaces 101 and 103. The probe head 101 is a convex-type. The probe head 103 is a linear-type. A ring-shaped pressing bag 151 is provided so as to cover only the outer circumference of the ultrasonic wave transmitting and receiving surface 101 that serves as a probe head section. The ring-shaped pressing bag 151 is structured so that the tip of the probe head can be inserted. The pressing bag 151 is inflated and deflated by liquid being fed to and removed from the pressing bag 151, thereby applying pressure to the tissue to be pressed. The above-described pump may be used as a power source used to feed the liquid into the pressing bag 151 and remove the liquid from the pressing bag 151, via a tube 152. Alternatively, the liquid may be manually fed to and removed from the pressing bag 151, using a syringe and the like. Although an example in which the ring-shaped pressing bag 151 is provided only on the outer circumference of the ultrasonic wave transmitting and receiving surface 101 is described, the pressing bag and the mechanism for the pressing bag may also be similarly provided for the outer circumference of the ultrasonic wave transmitting and receiving surface 103. By the pressing bags being provided on the outer circumference of the two ultrasonic wave transmitting and receiving surfaces, the surface from which the elasticity image is to be acquired can be selected and pressure can be applied thereto. At the same time, by the two pressing bags being simultaneously inflated, the elasticity images of two cross-sections may be simultaneously acquired.

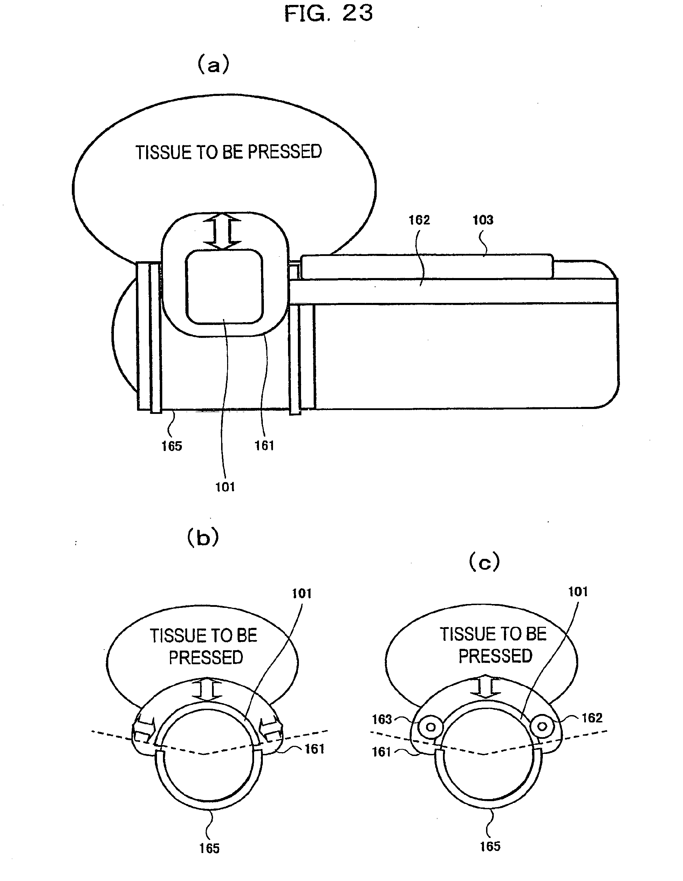

FIG. 23 is a diagram showing a modification of the automatic pressing mechanism 22 described in FIG. 22. The automatic pressing mechanism 22 is provided in the transrectal ultrasonic probe. FIG. 23(a) is a partially enlarged view. FIG. 23(b) is a diagram of the ultrasonic probe in FIG. 23(a) viewed from the left-hand side. FIG. 23(c) is a diagram of the ultrasonic probe in FIG. 23(a) viewed from the right-hand side. The ultrasonic probe includes two probe heads; i.e., the ultrasonic wave transmitting and receiving surfaces 101 and 103. The ring-shaped pressing bag 151 is provided so as to cover only the outer circumference of the ultrasonic wave transmitting and receiving surface 101 that serves as a probe head section. A stopper 165 that restricts the inflating direction of the pressing bag 161 is attached to the outside of the ring-shaped pressing bag 161. Since the direction of the inflation of the pressing bag 161 is restricted to one direction, pressure can be effectively applied to the inner surface of the rectum of the subject 1 by feeding liquid to the pressing bag 161 and removing the liquid therefrom. At this time, the surface of the stopper 165 serves as a supporting surface which comes into contacts with a surface area of the inner surface of the rectum of the subject 1, the surface area being opposite to a portion to be imaged. The above-described pump may be used as a power source used to feed the liquid into the pressing bag 161 and remove the liquid from the pressing bag 161, via tubes 162 and 163. According to an embodiment of the present invention, the liquid may be manually fed to and removed from the pressing bag 161, using a syringe and the like.

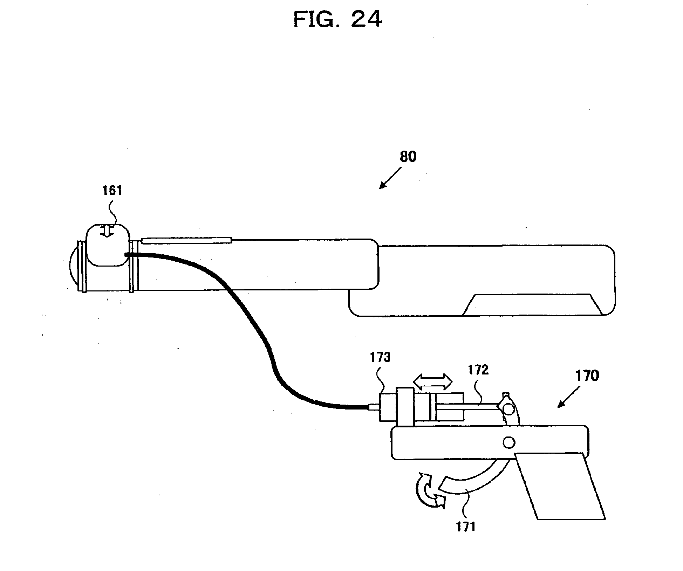

FIG. 24 to FIG. 28 are diagrams showing modifications of the power used to feed the liquid into the pressing bag and remove the liquid from the pressing bag. In the above-described examples, mechanical power from a motor mechanism, a pump, or the like being used as the power is described. However, these modifications employ manual operation structures which enable the operator to easily perform the operation of feeding and removing the liquid by use of one hand only. For example, a mechanism that can be operated manually, such as the trigger of a water pistol or the holding section of a grip dynamometer, is used as the power source in accordance with the present invention.

FIG. 24 is a diagram showing an application of the trigger section of a water pistol as the power source for feeding liquid to the pressing bag and removing the liquid therefrom. The water pistol section is provided separately from the ultrasonic probe. The end of a piston rod 172 is connected to a trigger section 171 of a water pistol 170. The piston of a syringe 173 is inserted and removed by the trigger section 171 being manually operated, thereby controlling the feed of the liquid to the pressing bag 161 and removal of the liquid therefrom. At this time, the reaction force of the liquid fed to the pressing bag 161 is transmitted to the trigger section 171. Therefore, the degree of pressing (pressure within the pressing bag 161) can be felt by the hand. Therefore, delicate pressing actions can be realized. Although the structure of FIG. 24 has been described with the pressing bag 161 in FIG. 23 taken as an example, the structure can be similarly applied to the above-described various pressing bags.

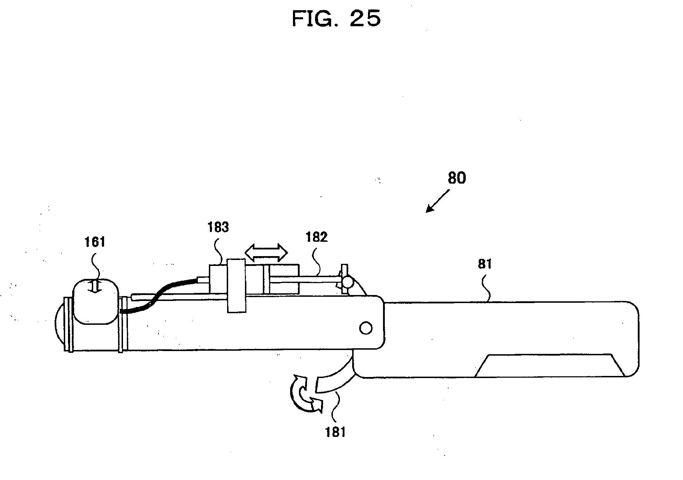

FIG. 25 is a modification of FIG. 24. A trigger section of the water pistol is again provided integrally with the ultrasonic probe and used as a power source for feeding liquid to the pressing bag and removing the liquid therefrom. A trigger section 181 is rotatably provided on the probe gripping section 81. A syringe 183 is provided on the outer circumference of the probe head. The end of a piston rod 182 is connected to a trigger section 181. The piston of a syringe 183 is inserted and removed by the trigger section 181 being manually operated, thereby controlling the feed of liquid to the pressing bag 161 and the removal of the liquid therefrom.

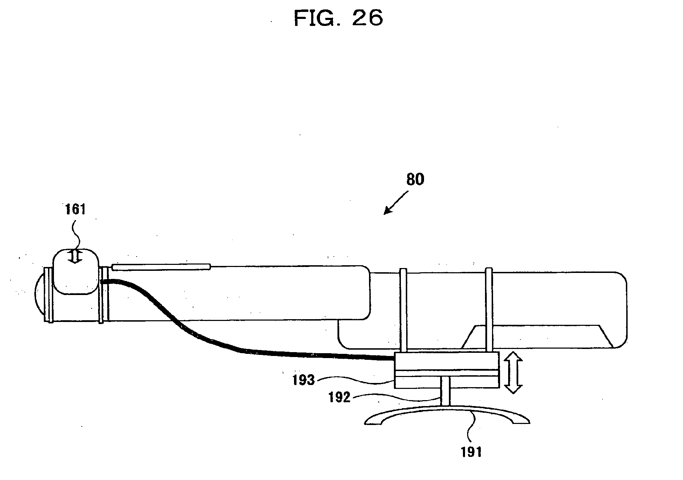

FIG. 26 is a modification of FIG. 25. In place of the trigger section of the water pistol, a structure like the holding section of a grip dynamometer is provided integrally with the ultrasonic probe and used as a power source for feeding and removing liquid from the pressing bag. A syringe 193 having a larger working area than the syringe used in FIG. 24 and FIG. 25 is provided on a side surface of the probe gripping section 81. A gripping section 191 having a structure similar to that of the holding section of the grip dynamometer is provided at the end of the piston rod 192. The gripping section 191 can be pressed by hands and fingers. The operator holds the probe gripping section 81, and applies a gripping force of the fingers to the gripping section 191 so as to insert and remove the piston of a syringe 193, thereby controlling the feed of liquid to the pressing bag 161 and the removal of the liquid therefrom.

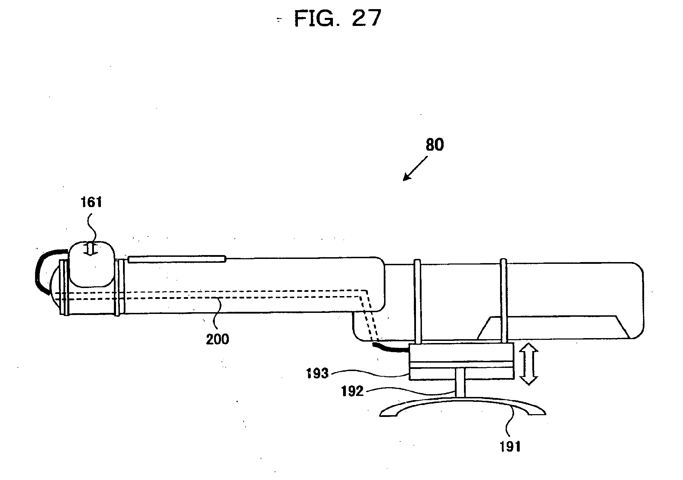

FIG. 27 is a variation example of FIG. 26. The tube from the syringe 193 to the pressing bag 161 is omitted. A pipe 200 incorporated in the handle of the probe that is originally attached to the ultrasonic probe is used. In FIG. 27, a tube is provided to connect the pipe 200 at the tip section of the ultrasonic probe and the pressing bag 161. However, the ultrasonic probe may be configured so that the pipe is curved within the ultrasonic probe to be connected directly to the pressing bag 161.

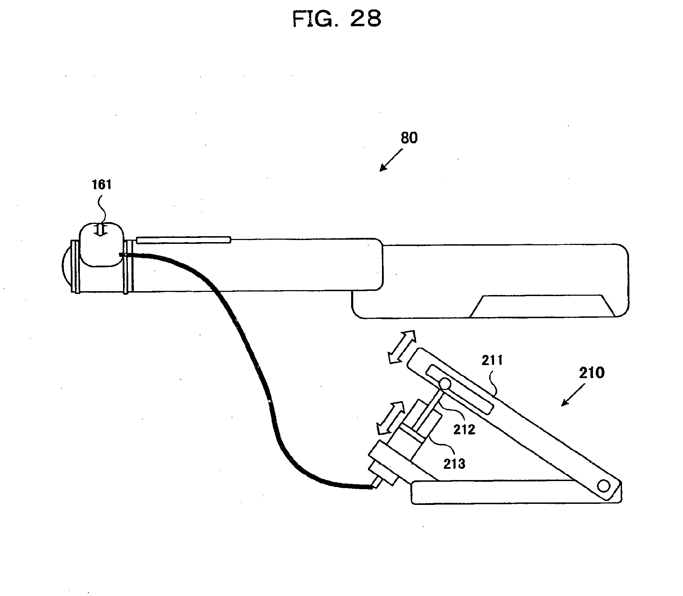

FIG. 28 is a modification of FIG. 24. In place of the water pistol 170 that can be operated by the fingers, a foot-pedal-type power source that can be operated by a foot is used. The foot-pedal-type power source 210 is provided separately from the ultrasonic probe and can be operated by foot. This is advantageous in that both hands may be used freely. In the foot-pedal-type power source 210, the end of a piston rod 212 is connected to a pedal section 211. The piston of a syringe 213 is inserted and removed by the pedal section 211 being operated by foot, thereby controlling the feed of liquid to the pressing bag 161 and the removal of the liquid therefrom. At this time, the reaction force of the liquid fed to the pressing bag 161 is transmitted to the pedal section 211. The degree of pressing (pressure within the pressing bag 161) can be felt by the foot. Therefore, delicate pressing actions can be realized. A stopper for preventing stepping can be provided on the rotatable section of the pedal 211 to prevent the pedal 211 from being excessively stepped.

Although the embodiments of FIG. 24 to FIG. 28 have been described with the pressing bag 161 taken as an example, these embodiments can be similarly applied to the above-described various pressing bags. Cylinder operating sections that are fixed to the probe or are separate from the ultrasonic probe are described in the above-described embodiments. However, the cylinder operating section may be configured such that it can be attached and removed in an attachment fashion, as long as the operator can easily operate the cylinder by use of the hand holding the ultrasonic probe. When the hydraulic pressure measured in this way is higher than a certain threshold, a warning of the danger and the like may be fed back by screen display, sound, or the like.

As described above, according to the embodiments, the following effects can be achieved. Image diagnosis having a high repeatability can be realized without dependence on technique. The subject can be uniformly pressed at a wide-angle and misdiagnosis due to lack of pressing can be prevented. The strength of the pressing can be fed back, and a safe diagnosis can be performed.

In the intra-corporeal ultrasonic probe described above, a pressure measuring section 21 (refer to FIG. 1) may be configured as follows. In the pressure measuring section 21, pressure sensors are provided on the periphery of the ultrasonic wave transmitting and receiving surface 101, as described in FIG. 7, whereby the pressure measuring section 21 measures the pressure received by the subject 1 from the pressing surface and outputs the pressure data. The pressure measuring section 21 may acquire pressure data by measuring the load acting on the driving mechanism of the automatic pressing mechanism 22 and calculating the pressure that the subject receives from the pressing surface, on the basis of the load.

When the automatic pressing mechanism 22 includes the pressing bag and the tube, the pressure measuring section 21 may acquire pressure data by measuring the internal pressure within the pressing bag or the tube. An example in which the pressure measuring section 21 measures the pressure received by the skin of the subject 1 and controls the operation of the automatic pressing mechanism 22 using the pressure data will be described. That is, the pressure measuring section 21 measures the pressure applied to the skin of the subject 1 by measuring the fluid pressure within the pressing bag. In this way, the pressure between the pressure place 101 and the skin of the subject 1 is measured at an arbitrary time, and the pressure data is outputted to the automatic pressing mechanism 22 and the distortion and elastic modulus calculating section 23. In other words, the automatic pressing mechanism 22 acquires the pressure data measured by the pressure measuring section 21 and controls the pressing actions of the automatic pressing mechanism 22 according to the pressure data.

There will be described the case where, as shown in FIG. 1, the automatic pressing mechanism 22 and the pressure measuring section 21 are connected together in order to control the operation of the automatic pressing mechanism 22. A pressing mechanism that uses driving power generated by the motor mechanism 41 has been shown as an example of the above-described automatic pressing mechanism 22. However, the motor control section of the automatic pressing mechanism 22 may be controlled based on the pressure data acquired by the liquid pressure within the pressing bag being measured. The motor control section outputs the motor control signal, based on the measured pressure data, to the motor mechanism 41 and controls the motor mechanism 41 so that the motor mechanism 41 performs the desired pressing action.

Through the use of the automatic pressing mechanism 22, the operation of the automatic pressing mechanism 22 can be stopped when the pressure measuring section 21 measures a pressure higher than a certain reference level. As a result, excessive pressure is not applied to the subject. In addition, when capturing the elasticity image, there is a pressure range within which a high-quality elasticity image can be obtained. When pressure exceeding the upper limit or pressure below the lower limit is applied, it is known that the elasticity image deteriorates.

In the automatic pressing mechanism 22, when the pressure measuring section 21 measures a pressure higher than a certain threshold during a certain continuous pressure increasing process, the operation of the automatic pressing mechanism 22 can be controlled so that the pressure increasing process is switched to the continuous pressure decreasing process. On the other hand, when the pressure measuring section 21 measures a pressure lower than a certain threshold, the operation of the automatic pressing mechanism 22 can be controlled so that the pressure decreasing process is switched to the continuous pressure increasing process. Through the repetition of this operation, an appropriately pressed state can be always maintained. As a result, a high-quality elasticity image can be efficiently acquired during a limited imaging time.

The amount of fluid flowing in and flowing out with one stroke of the syringe can be adjusted freely. In addition, in the intra-corporeal ultrasonic probe, when the actuator changes the distance between the probe gripping section 81 and the ultrasonic wave transmitting and receiving surface 101, pressure can be applied to the subject without the supporting surface coming in contact with a surface of the subject opposite the portion to be imaged.

Through the use of the automatic pressing mechanism 22 , pressure may be automatically applied to the subject at a desired fixed speed in a fixed direction. Therefore, high-quality elasticity image data can be acquired at an arbitrary time. Furthermore, the repeatability of the pressing action can be maintained.

The automatic pressing mechanism acquires information on the interval between the pair of ultrasonic wave reception signal frame data N and X, selected by the ultrasonic wave reception signal frame data selecting section 19, and controls the pressing action of the automatic pressing mechanism 22 based on the interval. Hereafter, an example of this operation will be described.

An operation of an ultrasonic imaging device, configured as described above, will be described. First, under ultrasonic wave transmission and reception control, the wave transmission circuit 12 applies a high-voltage electrical pulse to the ultrasonic probe 10 that is brought into contact with the surface of the body of the subject, thereby generating the ultrasonic waves. The ultrasonic probe 10 receives the reflection echo signal from the to-be-imaged portion. Next, the reception signal is inputted to the reception circuit 13 and pre-amplified. Then, the pre-amplified signal is inputted to the phase adjustment/addition circuit 14. The reception signal whose phase has been adjusted by the phase adjustment/addition circuit 14 is subjected to signal processing such as compression and wave detection in the next signal processing section 15. Then, the signal is inputted to the black-and-white scan converter 16. In the black-and-white scan converter 16, the reception signal is subjected to an analog-to-digital conversion, and is stored in a plurality of internal frame memories, as a plurality of temporally consecutive tomographic data.

In order to evaluate the elasticity of a portion of interest in the internal tissues of the subject by use of the ultrasonic probe 10 including the automatic pressing mechanism 22, the ultrasonic probe 10 is brought into contact with the body surface of the subject, such that the subject is pressed by the automatic pressing mechanism 22 in accordance with an automatically-set, appropriate pressing method, whereby consecutive ultrasonic wave reception signal frame data are outputted from the phase adjustment/addition circuit 14. The consecutive ultrasonic wave reception signal frame data outputted from the phase adjustment/addition circuit 14 are sequentially stored in the ultrasonic wave reception signal frame data selecting section 19. Of the stored ultrasonic wave reception signal frame data, a plurality of temporally consecutive ultrasonic wave reception signal frame data are selected by the ultrasonic wave reception signal frame data selecting section 19, and fed to the displacement measuring section 20. The displacement measuring section 20 determines a one-dimensional or two-dimensional displacement distribution ($\Delta Li,j$). The calculation of the displacement distribution is performed by the above-described methods for detecting motion vectors, such as the block matching method. It goes without saying that the above-described methods for detecting motion vectors do not have to be used in particular. The displacement may be calculated by the generally-used calculation of autocorrelation in the same regions of two image data. In addition, the information on the interval between the pair of ultrasonic wave reception signal frame data selected by the ultrasonic wave reception signal frame data selecting section is outputted to the automatic pressing mechanism 22. Based on the interval information, the pressing action of the automatic pressing mechanism 22 is optimized. At the same time, the pressure measuring section 21 measures the pressure applied to the body surface by pressure sensors. The pressure measuring section 21 sends the pressure information to the distortion and elastic modulus calculating section 23 and the automatic pressing mechanism 22. Based on the pressure information, the pressing action of the automatic pressing mechanism 22 is optimally controlled. As a result, elasticity image diagnosis of a subject can be performed efficiently and safely.

Next, the respective measurement signals of the displacement ($\Delta$Li,j) and pressure ($\Delta$Pi,j) outputted from the displacement measuring section 20 and the pressure measuring section 21 are inputted to the distortion and elastic modulus calculating section 23. The distortion and elastic modulus calculating section 23 spatially differentiates ($\Delta$Li,j/$\Delta$x) the displacement distribution ($\Delta$Li,j) and calculates the distortion amount distribution ($\epsilon$i,j). Among the elastic modulus, the Young's modulus of elasticity Ymi,j is calculated by the following equation:

$$\mathtt{Ymi,j=(\Delta Pi,j)/(\Delta Li,j/\Delta X)}$$

Based on the elastic modulus Ymi,j determined in this way, the elastic modulus at each point is determined. Thereby, two-dimensional elasticity image data is consecutively acquired.

Next, the elasticity frame data obtained in this way is inputted to the color scan converter 25 or the black-and-white scan converter 16 and is converted to color information or black-and-white brightness information. Then, the switching/adding unit 17 adds and composites the black-and-white tomographic image and the colored elasticity image and outputs the composite image to the image display unit 18. Alternatively, the switching/adding unit 17 outputs the black-and-white tomographic image and the black-and-white elasticity image to the image display unit 18 without adding the images. In the image display unit 18, the black-and-white tomographic image and the colored elasticity image are superposed and displayed on one screen. Alternatively, the black-and-white tomographic image and the black-and-white elasticity image may be simultaneously displayed on the same screed by a two-screen display. The black-and-white tomographic image is not particularly limited to the typical B-mode image. The black-and-white tomographic image may be a tissue harmonic tomographic image. In the tissue harmonic tomographic image, high-harmonic components of the reception signal are selected and converted to images. Similarly, a tissue Doppler image may be displayed in place of the black-and-white tomographic image. Furthermore, images to be displayed in the two-screen display may be selected in various combinations.

With regards to the form of the elasticity image, an example in which the distortion or Young's modulus of elasticity Ym of the biological tissue is obtained and the elasticity image data is generated is described. However, the present invention is not limited to this example. The elastic modulus may be calculated using other parameters, such as the stiffness parameter $\beta$, the pressure-elasticity coefficient Ep, and the incremental elasticity coefficient Einc (see Patent Document 1).

The above-described embodiment can be applied to an arbitrary intra-corporeal ultrasonic probe, such as the transrectal ultrasonic probe, the transesophageal ultrasonic probe, and the endovascular ultrasonic probe.

In this way, in the ultrasonic imaging device according to the embodiments, a high-quality elasticity image having a high repeatability and that is not dependent on the technician can be easily and safely acquired. In addition, in the ultrasonic imaging device according to the embodiments, the elasticity image can be stably extracted at a high-resolution at an arbitrary time. Furthermore, in the ultrasonic imaging device according to the embodiments, a means for visually expressing, by use of a dynamic image, a response of an examination by touch, conventionally attempted by doctors, is realized, whereby a clinically useful ultrasonic imaging device which enables convenient, real-time ultrasonic diagnosis can be provided.

Preferred embodiments of the ultrasonic probe and the ultrasonic imaging device according to the present invention have been described with reference to the accompanying drawings. However, the present invention is not limited to the above-described embodiments. Numerous modifications and variations of the present invention are possible within the technical scope of the present invention as defined in the appended claims.

Claims

1. An intra-corporeal ultrasonic probe (80) adapted to be inserted into a subject (1), comprising:

   a pressing member (161) provided on the probe (80) and having a contact surface (101) which extends in parallel to an insertion direction and which is adapted to come into contact with the subject (1); and
   pressing means for pressing the pressing member (161) against a to-be-imaged portion of the subject (1) with a predetermined pressure at the contact surface (101) in a direction perpendicular to the contact surface (101) by feeding liquid to the pressing member (161) and removing the liquid therefrom,
**characterized in that** the pressing means includes a syringe (173; 183; 193; 213) and a trigger section (171; 181; 191; 211) operated manually or by foot to insert and remove a piston rod (172; 182; 192; 212) of the syringe (173; 183; 193; 213) thereby controlling the feed of the liquid to the pressing member (161) and removal of the liquid therefrom, wherein the reaction force of the liquid fed to the pressing member (161) is transmitted to the trigger section (171; 181; 191; 211) so that the degree of pressing of the pressing member (161) can be felt by the hand or foot operating the trigger section (171; 181; 191; 211).

2. The probe of claim 1, wherein the pressing means is removably attached to an existing intra-corporeal ultrasonic probe (80).

3. The probe of any preceding claim, wherein the pressing member (161) is connected to the contact surface (101).

4. The probe of any preceding claim, further comprising a stopper (165) for restricting the inflating direction of the pressing member (161).

5. The probe of any preceding claim, wherein at least a portion of the pressing member (161) has a stretchability smaller than that of the remaining portion, so that the pressing member (161) is adapted to inflate and deflate while deforming in a predetermined direction.

6. An intra-corporeal ultrasonic imaging device comprising:

   the intra-corporeal ultrasonic probe (80) of any preceding claim;
   ultrasonic wave transmitting and receiving means (12, 13) for transmitting ultrasonic waves to a subject (1) and receiving ultrasonic waves from the subject (1) by use of the probe (80);
   ultrasonic wave transmission and reception control means (11) for controlling the transmission and reception of the ultrasonic waves;
   tomographic scanning means for repeatedly acquiring, at predetermined intervals, ultrasonic wave reception signal frame data representing an image of the interior of the subject (1), including a locomotive tissue, by use of a reflection echo signal output from the ultrasonic wave transmitting and receiving means (12, 13);
   signal processing means (15) for performing signal processing on a plurality of time-series ultrasonic wave reception signal frame data acquired by the tomographic scanning means;
   black-and-white brightness information converting means (16) for converting the time-series tomographic frame data from the signal processing means (15) to black-and-white tomographic data;
   ultrasonic wave reception signal frame data selecting means (19) for selecting a pair of ultrasonic wave reception signal frame data to be subjected to displacement measurement, among the group of time-series ultrasonic wave reception signal frame data acquired by the tomographic scanning means;
   displacement measuring means (20) for measuring the amount of movement or displacement of each point on a tomographic image, based on the pair of ultrasonic wave reception signal frame data selected by the ultrasonic wave reception signal frame data selecting means (19);
   pressure measuring means (21) for measuring or estimating an internal pressure of the to-be-imaged portion of the subject (1);
   distortion and elastic modulus calculating means (23) for calculating a distortion and an elastic modulus at each point on the tomographic image, based on the displacement measured by the displacement measuring means (20) and the internal pressure measured by the pressure measuring means (21), and for generating a first elasticity frame data;
   elasticity data processing means (24) for performing signal processing on the first elasticity frame data generated by the distortion and elastic modulus calculating means (23) and for generating a second elasticity frame data;
   color information converting means (25), or black-and-white brightness information converting means, for receiving the second elasticity frame data generated by the elasticity data processing means (24), and for providing color information, or black-and-white brightness information;
   switching-adding means (17) for receiving the black-and-white tomographic image data from the black-and-white brightness information converting means (16) and the colored elasticity image data from the color information converting means (25) or the black-and-white elasticity image data from the black-and-white brightness information converting means (16) and for outputting them after adding them together or outputting them independently; and
   image displaying means (18) for displaying the image data from the switching-adding means (17).

**Patentansprüche**

1. Intrakorporale Ultraschallsonde (80), die zur Einführung in ein Subjekt (1) ausgelegt ist, mit einem Druckelement (161), das auf der Probe (80) vorgesehen ist und eine Kontaktfläche (101) aufweist, die parallel zu einer Einführrichtung verläuft und dazu ausgelegt ist, in Kontakt mit dem Subjekt (1) zu kommen, und einer Druckeinrichtung zum Drücken des Druckelements (161) gegen einen abzubildenden Abschnitt des Subjekts (1) mit einem vorbestimmten Druck an der Kontaktfläche (101) in einer Richtung senkrecht zu der Kontaktfläche (101), indem eine Flüssigkeit zu dem Druckelement (161) zugeführt wird und die Flüssigkeit davon entfernt wird, **dadurch gekennzeichnet, dass** die Druckeinrichtung eine Spritze (173; 183; 193; 213) und einen Auslöseabschnitt (171; 181; 191; 211) aufweist, der manuell oder per Fuß betrieben wird, um einen Kolbenstab (172; 182; 192; 212) der Spritze (173; 183; 193; 213) einzuschieben und zu entfernen, um dadurch die Zufuhr der Flüssigkeit zu dem Druckelement (161) und das Entfernen der Flüssigkeit davon zu steuern, wobei die Reaktionskraft der zu dem Druckelement (161) zugeführten Flüssigkeit zu dem Auslöseabschnitt (171; 181; 191; 211) übertragen wird, so dass die Druckstärke des Druckelements (161) durch die Hand oder den Fuß gefühlt werden kann, die den Auslöseabschnitt (171; 181; 191; 211) betätigt.

2. Sonde nach Anspruch 1, wobei die Druckeinrichtung entfernbar an einer existierenden intrakorporalen Ultraschallsonde (80) angebracht ist.

3. Sonde nach einem der vorstehenden Ansprüche, wobei das Druckelement (161) mit der Kontaktfläche (101) verbunden ist.

4. Sonde nach einem der vorstehenden Ansprüche, ferner mit einem Stoppelement (165) zum Begrenzen der Aufweitrichtung des Druckelements (161).

5. Sonde nach einem der vorstehenden Ansprüche, wobei wenigstens ein Abschnitt des Druckelements (161) eine geringere Dehnbarkeit aufweist als der übrige Abschnitt, so dass das Druckelement (161) dazu ausgelegt ist, sich aufzuweiten und zusammenzuziehen, während es in einer vorbestimmten Richtung deformiert wird.

6. Intrakorporale Ultraschallabbildungsvorrichtung mit der intrakorporalen Ultraschallsonde (80) nach einem der vorstehenden Ansprüche, einer Ultraschallwellen-Sende/Empfangs-Einrichtung (12, 13) zum Senden von Ultraschallwellen zu einem Subjekt (1) und Empfangen von Ultraschallwellen von dem Subjekt (1) unter Verwendung der Sonde (80), einer Ultraschallwellen-Sende/Empfangs-Steuereinrichtung (11) zum Steuern des Sendens und Empfangens der Ultraschallwellen, einer Tomographieabtasteinrichtung zum wiederholten Erhalten, zu vorbestimmten Zeitabschnitten, von Ultraschallwellenempfangssignalrahmendaten, die ein Bild des Inneren des Subjekts (1), einschließlich des lokomotorischen Gewebes, darstellen, indem ein Reflexionsechosignal verwendet wird, das von der UI-traschallwellen-Sende/Empfangs-Einrichtung (12, 13) ausgegeben wird, einer Signalverarbeitungseinrichtung (15) zum Durchführen von Signalverarbeitung an mehreren zeitreihenbasierten Ultraschallwellenempfangssignalrahmendaten, die durch die Tomographieabtasteinrichtung erhalten wurden, einer Schwarz-und-Weiß-Helligkeitsinformations-Konvertierungseinrichtung (16) zum Konvertieren der zeitreihenbasierten tomographischen Rahmendaten von der Signalverarbeitungseinrichtung (15) in Schwarz-und-Weiß-Tomographiedaten, einer Ultraschallwellenempfangssignalrahmendaten-Auswahleinrichtung (19) zum Auswählen eines Paars von einer Verschiebungsmessung zu unterziehenden Ultraschallwellenempfangssignalrahmendaten aus der Gruppe von zeitreihenbasierten Ultraschallwellenempfangssignalrahmendaten, die durch die Tomographieabtasteinrichtung erhalten wurden, einer Verschiebungsmesseinrichtung (20) zum Messen des Bewegungs- oder Verschiebungsbetrags jedes Punkts auf einem Tomographiebild, basierend auf dem Paar von Ultraschallwellenempfangssignalrahmendaten, das durch die Ultraschallwellenempfangssignalrahmendaten-Auswahleinrichtung (19) ausgewählt wurde, einer Druckmesseinrichtung (21) zum Messen oder Einschätzen eines Innendrucks des abzubildenden Abschnitts des Subjekts (1), einer Verzerrungs- und Elastizitätsmodul-Berechnungseinrichtung (23) zum Berechnen einer Verzerrung und eines Elastizitätsmoduls an jedem Punkt auf dem Tomographiebild, basierend auf der durch die Verschiebungsmesseinrichtung (20) gemessenen Verschiebung und des durch die Druckmesseinrichtung (21) gemessenen Innendrucks, und zum Erzeugen von ersten Elastizitätsrahmendaten, einer Elastizitätsdatenverarbeitungseinrichtung (24) zum Durchführen von Signalverarbeitung an den durch die Verzerrungs- und Elastizitätsmodul-Berechnungseinrichtung (23) erzeugten ersten Elastizitätsrahmendaten und zum Erzeugen von zweiten Elastizitätsrahmendaten,

einer Farbinformations-Konvertierungseinrichtung (25) oder einer Schwarz- und-Weiß-Helligkeitsinformations-Konvertierungseinrichtung zum Empfangen der durch die Elastizitätsdatenverarbeitungseinrichtung (24) erzeugten zweiten Elastizitätsrahmendaten und zum Bereitstellen von Farbinformation oder Schwarz- und-Weiß-Helligkeitsinformation,

einer Umschalt-Addier-Einrichtung (17) zum Empfangen der Schwarz-und-Weiß-Tomographiebilddaten von der Schwarz-und-Weiß-Helligkeitsinformations-Konvertierungseinrichtung (16) und der farbigen Elastizitätsbilddaten von der Farbinformations-Konvertierungseinrichtung (25) oder der Schwarz-und-Weiß-Elastizitätsbilddaten von der Schwarz-und-Weiß-Helligkeitsinformations-Konvertierungseinrichtung (16) und zum Ausgeben derselben nach deren Addition oder unabhängig voneinander, und

einer Bildanzeigeeinrichtung (18) zum Anzeigen der Bilddaten aus der Umschalt-Addier-Einrichtung (17).

## Revendications

**1.** Sonde à ultrasons intracorporelle (80) adaptée pour être insérée dans un sujet (1), comprenant :

un élément de pression (161) placé sur la sonde (80) et ayant une surface de contact (101) qui s'étend parallèlement à une direction d'insertion et qui est adaptée pour venir en contact avec le sujet (1) ; et

un moyen de pression pour pousser l'élément de pression (161) contre une partie du sujet (1) dont on veut prendre une image avec une pression prédéterminée au niveau de la surface de contact (101) dans une direction perpendiculaire à la surface de contact (101) en introduisant un liquide dans l'élément de pression (161) et en retirant le liquide de celui-ci,

**caractérisée en ce que** le moyen de pression comprend une seringue (173 ; 183 ; 193 ; 213) et une section de déclenchement (171 ; 181 ; 191 ; 211) actionnée manuellement ou au pied pour insérer et retirer une tige de piston (172 ; 182 ; 192 ; 212) de la seringue (173 ; 183 ; 193 ; 213), ce qui commande l'envoi de liquide vers l'élément de pression (161) et le retrait du liquide de ce dernier, dans laquelle la force de réaction du liquide envoyé à l'élément de pression (161) est transmise à la section de déclenchement (171 ; 181 ; 191 ; 211) de telle manière que le degré de pression de l'élément de pression (161) peut être senti par la main ou le pied qui actionne la section de déclenchement (171 ; 181 ; 191 ; 211).

**2.** Sonde selon la revendication 1, dans laquelle le moyen de pression est fixé de manière amovible à une sonde à ultrasons intracorporelle existante (80).

**3.** Sonde selon l'une quelconque des revendications précédentes, dans laquelle l'élément de pression (161) est connecté à la surface de contact (101).

**4.** Sonde selon l'une quelconque des revendications précédentes, comprenant en outre un élément d'arrêt (165) pour limiter la direction de gonflage de l'élément de pression (161).

**5.** Sonde selon l'une quelconque des revendications précédentes, dans laquelle au moins une partie de l'élément de pression (161) a une extensibilité inférieure à celle de la partie restante, de sorte que l'élément de pression (161) est adapté pour se gonfler et se dégonfler tout en se déformant dans une direction prédéterminée.

**6.** Dispositif d'imagerie intracorporel à ultrasons comprenant :

une sonde à ultrasons intracorporelle (80) selon l'une quelconque des revendications précédentes ;

un moyen d'émission et de réception d'ondes ultrasonores (12, 13) pour émettre des ondes ultrasonores vers un sujet (1) et recevoir des ondes ultrasonores du sujet (1) par l'intermédiaire de la sonde (80) ;

un moyen de commande d'émission et de réception d'ondes ultrasonores (11) pour commander l'émission et la réception des ondes ultrasonores ;

un moyen de balayage tomographique pour acquérir de façon répétée, à des intervalles prédéterminés, des données de trame de signal de réception d'ondes ultrasonores représentant une image de l'intérieur du sujet (1), comportant un tissu locomoteur, en utilisant un signal d'écho de réflexion délivré par le moyen d'émission et de réception d'ondes ultrasonores (12, 13) ;

un moyen de traitement du signal (15) pour réaliser un traitement de signal sur une pluralité de données de trame de signal de réception d'ondes ultrasonores de séries chronologiques acquises par le moyen de balayage tomographique ;

un moyen de conversion d'information de luminosité noir et blanc (16) pour convertir les données de trame tomographique de séries chronologiques provenant du moyen de traitement du signal (15) en données tomographiques noir et blanc ;

un moyen de sélection de données de trame de signal de réception d'ondes ultrasonores (19) pour sélectionner une paire de données de trame de signal de réception d'ondes ultrasonores à soumettre à une mesure de déplacement, parmi le groupe de données de trame de signal de réception d'ondes ultrasonores de séries chronologiques acquises par le moyen de balayage tomographique ;

un moyen de mesure de déplacement (20) pour mesurer la quantité de mouvement ou de déplacement de chaque point sur une image tomographique, en se basant sur la paire de données de trame de signal de réception d'ondes ultrasonores sélectionnée par le moyen de sélection de données de trame de signal de réception d'ondes ultrasonores (19) ;

un moyen de mesure de pression (21) pour mesurer ou estimer une pression interne de la partie du sujet (1) dont on veut prendre une image ;

un moyen de calcul de distorsion et de module d'élasticité (23) pour calculer une distorsion et un module d'élasticité en chaque point de l'image tomographique, d'après le déplacement mesuré par le moyen de mesure de déplacement (20) et la pression interne mesurée par le moyen de mesure de pression (21), et pour générer une première donnée de trame d'élasticité ;

un moyen de traitement de données d'élasticité (24) pour réaliser un traitement de signal sur la première données de trame d'élasticité générée par le moyen de calcul de distorsion et de module d'élasticité (23) et pour générer une deuxième donnée de trame d'élasticité ;

un moyen de conversion d'information de couleur (25), ou un moyen de conversion d'information de luminosité noir et blanc, pour recevoir la deuxième donnée de trame d'élasticité générée par le moyen de traitement de données d'élasticité (24), et pour fournir une information de couleur, ou une information de luminosité noir et blanc ;

un moyen d'ajout et de commutation (17) pour recevoir les données d'image tomographique noir et blanc du moyen de conversion d'information de luminosité noir et blanc (16) et les données d'image d'élasticité colorée provenant du moyen de conversion d'information de couleur (25) ou les données d'image d'élasticité noir et blanc du moyen de conversion d'information de luminosité noir et blanc (16) et pour les délivrer en sortie après les avoir additionnées ou pour les délivrer indépendamment ; et

un moyen d'affichage d'image (18) pour afficher les données d'image provenant du moyen d'ajout et de commutation (17).

FIG. 1

FIG. 2

# FIG. 3

FIG. 4

FIG. 5

## FIG. 6

FIG. 7

FIG. 8

FIG. 9

# FIG. 10

# FIG. 11

(b)

(a)

# FIG. 12

(b)

D
E  82  B
102
F  A
83A  83E
83B  83D
83C

(a)

102
53A~53E
82
83A~83E
52A~52E

FIG. 13

(b)

(a)

# FIG. 14

(b)                    (a)

**FIG. 15**

(b)

(a)

FIG. 16

(b)

(a)

86

(c)

55

52

FIG. 17

TISSUE TO BE PRESSED

33
34
55
101
82
85

FIG. 18

FIG. 19

# FIG. 20

## (a)

TISSUE TO BE PRESSED

131

103

101

132

## (b)

101

TISSUE TO BE
PRESSED

131

## (c)

101

TISSUE TO BE
PRESSED

131

132

# FIG. 21

## (a)

TISSUE TO BE PRESSED

103

101

142

## (b)

TISSUE TO BE
PRESSED

101

141   141

## (c)

TISSUE TO BE
PRESSED

101

142

# FIG. 22

(a)

TISSUE TO BE PRESSED

103

101

152

(b)

101

TISSUE TO BE
PRESSED

151

(c)

101

TISSUE TO BE
PRESSED

151

152

# FIG. 23

## (a)

TISSUE TO BE PRESSED

162    103

165    161

101

## (b)

101

TISSUE TO BE
PRESSED

161

165

## (c)

101

TISSUE TO BE
PRESSED

163    162

161

165

FIG. 24

# FIG. 25

FIG. 26

FIG. 27

FIG. 28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEISEI5317313 B
- JP 2000060853 A
- US 6511427 B
- US 2002068870 A1
- EP 1629777 A1
- JP 6169922 A

**Non-patent literature cited in the description**

- **SHIINA.** Real time tissue elasticity imaging using the combined auto-correlation method. *Journal of Medical Ultrasonics, Japan Society of Ultrasonics in Medicine,* vol. 29, 119-128